# EUROPÄISCHE PATENTANMELDUNG

(11) **EP 4 141 775 A1**
(43) Veröffentlichungstag der Anmeldung: **01.03.2023**
(21) Anmeldenummer: 21193679.4
(22) Anmeldetag: 28.08.2021
(51) Int. Cl.: G06Q 30/06, G06T 7/62, A61B 5/107, A61B 5/00

(54) **VERFAHREN UND SYSTEM ZUM AUSWÄHLEN EINES AUTOINTERIEURPRODUKTS**

(71) Anmelder: iControl automotive GmbH, 82024 Taufkirchen (DE)
(72) Erfinder: Hild, Tobias, 82024 Taufkirchen (DE)
(74) Vertreter: Koelle, Alexander

(57) **Zusammenfassung**

Die vorliegenden Anmeldung betrifft ein Verfahren (30) zum Auswählen zumindest eines Autointerieurprodukts aus einer Vielzahl von in einer Datenbank hinterlegten Autointerieurprodukten für einen Autofahrer (4), umfassend die folgenden Verfahrensschritte:
Eingeben (30-1) von zumindest einem Eingabeparameter, insbesondere Autotyp, Einsatzbereich des Autos, gewünschte Härte der Sitzpolsterung, und/oder anatomische Besonderheiten des Autofahrers (4) durch eine Eingabevorrichtung (9) eines Mobilgeräts (6),
Aufnehmen (30-2) eines Fotos (14) von zumindest einem Körperteil des Autofahrers (4) durch eine Kamera (7) des Mobilgeräts (6),
Bestimmen (30-3) zumindest eines Körperparameters des Autofahrers (4) auf Basis des aufgenommenen Fotos (14) durch eine Steuerung (8-1),
Bestimmen (30-4) jeweils einer Produkteignung einer Mehrzahl von Autointerieurprodukten der Vielzahl von in der Datenbank hinterlegten Autointerieurprodukten in Abhängigkeit des bestimmten Körperparameters und in Abhängigkeit des zumindest einen eingegebenen Eingabeparameters durch die Steuerung (8-1),
Bestimmen (30-5) einer priorisierten Liste der Mehrzahl von Autointerieurprodukten durch die Steuerung (8-1), wobei in der priorisierten Liste die Priorität der jeweiligen Autointerieurprodukte mit zunehmender Produkteignung steigt, und
Ausgeben (30-6) einer Auswahl von zumindest einem Autointerieurprodukt für den Autofahrer (4) durch das Mobilgerät (6), wobei die Auswahl das zumindest eine Autointerieurprodukt der priorisierten Liste der Mehrzahl von Autointerieurprodukten umfasst, dessen jeweilige bestimmte Produkteignung eine geringste Differenz oder gar keine Differenz zu einer maximalen Produkteignung aufweist.

## Beschreibung

### Technisches Gebiet

Die vorliegende Anmeldung betrifft ein Verfahren und ein System zum Auswählen zumindest eines Autointerieurprodukts aus einer Vielzahl von in einer Datenbank hinterlegten Autointerieurprodukten für einen Autofahrer. Das Verfahren bzw. das System umfasst eine Kamera eines Mobilgeräts, durch welche ein Foto von zumindest einem Körperteil des Autofahrers aufgenommen wird, und umfasst eine Steuerung, durch welche auf Basis des aufgenommenen Fotos zumindest ein Körperparameter des Autofahrers bestimmt wird, wobei darauf basierend eine Produkteignung einer Mehrzahl von Autointerieurprodukten bestimmt wird, wobei darauf basierend eine priorisierte Liste der Mehrzahl von Autointerieurprodukten bestimmt wird, und wobei darauf basierend eine Auswahl von zumindest einem Autointerieurprodukt aus der Mehrzahl von Autointerieurprodukten für den Autofahrer durch das Mobilgerät ausgegeben wird.

### Stand der Technik

Bei der Auswahl eines individuell an einen Autofahrer angepassten Autointerieurprodukts, wie z.B. Autositz, insbesondere Polsterpad eines Autositzes, und/oder Autolenker, insbesondere Griffstück, besteht oftmals der Nachteil, dass entsprechende Körperparameter des Autofahrers durch einen Fachverkäufer zuerst aufwendig gemessen bzw. vermessen werden müssen. Anschließend müssen die gemessenen Körperparameter in Bezug auf das gewünschte Autointerieurprodukt manuell ausgewertet und dann einem an den Autofahrer optimal angepassten Autointerieurprodukt zugeordnet werden. Ein entsprechender Vorgang ist oftmals nicht nur mit einem hohen Zeit- und damit Kostenaufwand verbunden, sondern weist auch in Abhängigkeit von dem Fachverkäufer oftmals nur begrenzt zur Verfügung stehenden Detailinformationen hinsichtlich der relevanten Autointerieurprodukte eine hohe Ungenauigkeit bzw. Fehleranfälligkeit auf.

Aus dem Stand der Technik ist zwar beispielsweise für Fahrradprodukte bekannt, dass die Kontur des Gesäßes eines Fahrradfahrers, insbesondere der Sitzknochenabstand zwischen den beiden Sitzknochen des Fahrradfahrers, bei einer manuellen Sattelauswahl durch einen Fachverkäufer berücksichtigt werde kann, wie z.B. in der DE 20 2006 008 296 U1 bzw. EP 2 508 126 A1/B1, der EP 2 703 261 B1, der EP 3 290 887 A1 und der EP 3 395 658 A1 offenbart ist.

Ein Nachteil entsprechender herkömmlicher Sitzknochenabstandserfassungssysteme im Fahrradbereich ist unter anderem, dass zum Teil teure und komplexe Apparaturen benötigt werden, dass die Auswertung der entsprechend erfassten Gesäßabdrücke durch den Fachverkäufer oftmals aufwendig ist, dass die Gesäßabdrücke bzw. Sitzknochenabstände oftmals nicht oder nur unzureichend in digitalisierter Form gespeichert werden können, und dass die Zuordnung der Gesäßabdrücke bzw. Sitzknochenabstände, zu entsprechenden für den Fahrradfahrer optimalen Fahrradsätteln manuell durch den Fachverkäufer erfolgt und dadurch fehleranfällig sein kann.

Für eine individuelle Anpassung von Autointerieurprodukten, wie z.B. Autositz, insbesondere Polsterpad eines Autositzes, und/oder Autolenker, insbesondere Griffstück, ist jedoch aus dem Stand der Technik nicht bekannt, dass Körperparameter eines Autofahrers, wie z.B. Sitzknochenabstand, aber auch Schulterbreite, Kniehöhe, Hüftbreite, Schrittlänge und/oder Körpergröße, berücksichtigt werden.

Ferner ist aus dem Stand der Technik nicht bekannt, eine einfache und automatisierte Erfassung von individuellen Körperparametern eines Autofahrers, sowie eine entsprechende darauf basierende einfache und automatisierte Bestimmung von Produkteignungen entsprechender Autointerieurprodukten, sowie die sich daran anschließende Ausgabe einer Auswahl von einer für den jeweiligen Autofahrer optimalen Auswahl von Autointerieurprodukten durchzuführen, insbesondere automatisiert durchzuführen.

Insbesondere ist aus dem Stand der Technik nicht bekannt, dass eine einfache und automatisierte Erfassung von Körperparametern eines Autofahrers durch ein Mobilgerät bzw. durch eine auf dem Mobilgerät ausgeführte App, durchgeführt werden kann.

Auch ist aus dem Stand der Technik nicht bekannt, dass eine durch ein Mobilgerät durchgeführte einfache und automatisierte Erfassung entsprechender Körperparameter eines Autofahrers, zu einer für den Autofahrer optimalen Auswahl von Autointerieurprodukten führen kann.

### Offenlegung der Anmeldung

Die vorliegende Anmeldung stellt sich zur Aufgabe, ein Verfahren und ein System zur automatisierten und individualisierten Auswahl eines Autointerieurprodukts, insbesondere Autositz, Polsterpad eines Autositzes, und/oder Autolenker, insbesondere Griffstück eines Autolenkers, für einen Autofahrer bereitzustellen.

Die Aufgabe der Anmeldung wird gemäß einem ersten Aspekt durch ein Verfahren zum Auswählen zumindest eines Autointerieurprodukts für einen Autofahrer nach dem unabhängigen Anspruch 1, sowie gemäß einem zweiten Aspekt durch ein System zum Auswählen zumindest eines Autointerieurprodukts für einen Autofahrer nach dem unabhängigen Anspruch 15 gelöst. Die weiteren abhängigen Ansprüche beanspruchen bevorzugte Ausführungsformen.

Gemäß einem ersten Aspekt der vorliegenden Anmeldung wird die vorliegende Aufgabe durch ein Verfahren zum Auswählen zumindest eines Autointerieurprodukts aus einer Vielzahl von in einer Datenbank hinterlegten Autointerieurprodukten für einen Autofahrer gelöst, umfassend die folgenden Verfahrensschritte: Eingeben von zumindest einem Eingabeparameter, insbesondere Autotyp, Einsatzbereich des Autos, gewünschte Härte der Sitzpolsterung, und/oder anatomische Besonderheiten des Autofahrers durch eine Eingabevorrichtung eines Mobilgeräts, Aufnehmen eines Fotos von zumindest einem Körperteil des Autofahrers durch eine Kamera des Mobilgeräts, Bestimmen zumindest eines Körperparameters des Autofahrers auf Basis des aufgenommenen Fotos durch eine Steuerung, Bestimmen jeweils einer Produkteignung einer Mehrzahl von Autointerieurprodukten der Vielzahl von in der Datenbank hinterlegten Autointerieurprodukten in Abhängigkeit des bestimmten Körperparameters und in Abhängigkeit des zumindest einen eingegebenen Eingabeparameters durch die Steuerung, Bestimmen einer priorisierten Liste der Mehrzahl von Autointerieurprodukten durch die Steuerung, wobei in der priorisierten Liste die Priorität der jeweiligen Autointerieurprodukte mit zunehmender Produkteignung steigt, und Ausgeben einer Auswahl von zumindest einem Autointerieurprodukt für den Autofahrer durch das Mobilgerät, wobei die Auswahl das zumindest eine Autointerieurprodukt der priorisierten Liste der Mehrzahl von Autointerieurprodukten umfasst, dessen jeweilige bestimmte Produkteignung eine geringste Differenz oder gar keine Differenz zu einer maximalen Produkteignung aufweist.

Durch die Steuerung, insbesondere in Kombination mit einem auf dem Mobilgerät ablaufendem Programm bzw. App, kann somit auf eine einfache und automatisierte Weise eine Auswahl von individuell an den Autofahrer angepassten Autointerieurprodukten auf dem Mobilgerät ausgegeben werden, z.B. auf einem Display des Mobilgeräts.

Durch die Eingabevorrichtung des Mobilgeräts, insbesondere durch ein Touch-Display und/oder durch einen Touch-Button des Mobilgeräts, kann der Nutzer des Mobilgeräts zumindest einen der zur Autointerieurproduktbestimmung relevanten Eingabeparameter eingeben. Der zumindest eine Eingabeparameter umfasst insbesondere einen einzigen oder eine Mehrzahl der folgenden Eingabeparameter: den Autotyp, wie z.B. die Modellnummer eines Automodells eines Autoherstellers, wie z.B. Porsche 911, den Einsatzbereich des Autos, wie z.B. Rennstrecke, Langstrecke oder Kurzstrecke, die gewünschte Härte der Sitzpolsterung, wie z.B. sportlich straff, komfortabel weich, oder allround medium, und/oder anatomische Besonderheiten des Autofahrers, wie z.B. Probleme mit Halswirbel, Brustwirbel, Lendenwirbel, Knie, Hüfte, Schultergelenk und/oder Steißbein bzw. Verspannungen in Schulter und/oder Nacken, ein starkes Schwitzen des Autofahrers, und/oder einen besonderen Komfort der Sitzfläche.

Je nach gewünschtem Autointerieurprodukt kann auch nur eine Unterauswahl der insbesondere genannten Eingabeparameter durch die Eingabevorrichtung des Mobilgeräts eingegeben werden, da z.B. Probleme mit dem Steißbein des Autofahrers bei der Auswahl eines individuell an den Autofahrers angepassten Autolenkers unter Umständen irrelevant sein können.

Insbesondere umfasst das Eingeben des zumindest einen Eingabeparameters das Eingeben einer Mehrzahl von Eingabeparametern, wobei insbesondere die Reihenfolge der Eingaben der Eingabeparameter veränderbar ist.

Nach dem Eingeben kann der zumindest eine Eingabeparameter insbesondere hinterlegt werden, um z.B. bei einer späteren Autointerieurproduktauswahl für denselben Autofahrer einen raschen Zugriff auf zumindest einige der vormals eingegebenen Eingabeparameter zu ermöglichen.

Anschließend wird ein Foto von zumindest einem Körperteil des Autofahrers durch eine Kamera des Mobilgeräts aufgenommen. Insbesondere kann das zumindest eine Körperteil ein einziges Körperteil des Autofahrers umfassen, so dass z.B. für die Auswahl eines Autolenkers ein Foto einer Hand des Autofahrers aufgenommen wird oder so dass z.B. für die Auswahl eines Autositzes ein Foto des Gesäßbereichs des Autofahrers aufgenommen wird.

Es kann jedoch auch eine Mehrzahl von Körperteilen, insbesondere auch der gesamte Körper des Autofahrers, durch die Kamera des Mobilgeräts aufgenommen werden, so dass z.B. auf Basis der aufgenommenen Mehrzahl von Körperteile bzw. auf Basis des aufgenommenen gesamten Körpers des Autofahrers unterschiedlichen Autointerieurprodukte ausgegeben werden können.

Es kann jedoch auch ein Abbild zumindest eines Körperteils durch die Kamera des Mobilgeräts aufgenommen werden, wie z.B. ein Gesäßabdruck eines Gesäßes des Autofahrers, um auf der Basis es aufgenommenen Fotos eine vorteilhafte Ausgabe von Autointerieurprodukten, z.B. Autositzen, insbesondere Polsterpads von Autositzen, zu ermöglichen.

Das durch die Kamera aufgenommene Foto des zumindest einen Körperteils des Autofahrers kann ein einziges Foto oder eine Mehrzahl von Fotos umfassen, auf dessen Basis oder auf deren Basis anschließend der zumindest eine Körperparameter des Autofahrers bestimmt werden kann.

Der zumindest eine Körperparameter des Autofahrers wird auf Basis des durch die Kamera des Mobilgeräts aufgenommenen Fotos durch eine Steuerung bestimmt.

Die Steuerung kann die Steuerung des Mobilgeräts, insbesondere den Prozessor des Mobilgeräts, umfassen.

Alternativ kann die Steuerung Teil einer externen Datenverarbeitungseinrichtung sein, welche den zumindest einen Körperparameter des Autofahrers auf Basis des durch die Kamera des Mobilgeräts aufgenommenen Fotos bestimmt, berechnet oder sonstwie ermittelt. Hierbei weist das Mobilgerät insbesondere eine erste Kommunikationseinrichtung auf, und weist die externe Datenverarbeitungseinrichtung insbesondere eine zweite Kommunikationseinrichtung auf, so dass Daten von dem Mobilgerät, insbesondere das aufgenommene Foto, durch die erste Kommunikationseinrichtung und durch die zweite Kommunikationseinrichtung der externen Datenverarbeitungseinrichtung zur Verfügung gestellt werden können.

Der zumindest eine durch die Steuerung bestimmte Körperparameter umfasst insbesondere einen einzigen Körperparameter oder eine Mehrzahl von Körperparametern.

In Abhängigkeit des ausgewählten Autointerieurprodukts können unterschiedliche Körperparameter durch die Steuerung bestimmt werden. Soll beispielsweise eine Auswahl von Autositzen bzw. Polsterpads von Autositzen ausgegeben werden, wird insbesondere unter anderem der Sitzknochenabstand zwischen den beiden Sitzknochen des Gesäßes des Autofahrers als relevanter Körperparameter bestimmt. Soll beispielsweise eine Auswahl von Autolenkern bzw. Griffstücken von Autolenkern ausgegeben werden, wird insbesondere unter anderem eine Handbreite und/oder eine Handlänge einer Hand des Autofahrers als relevanter Körperparameter bestimmt.

Anschließend wird jeweils eine Produkteignung einer Mehrzahl von Autointerieurprodukten der Vielzahl von in der Datenbank hinterlegten Autointerieurprodukten in Abhängigkeit des bestimmten zumindest einen Körperparameters und in Abhängigkeit des zumindest einen eingegebenen Eingabeparameters durch die Steuerung bestimmt bzw. berechnet.

Hier kann die Datenbank und die Steuerung insbesondere Teil einer externen Datenverarbeitungseinrichtung sein, durch weiche das entsprechende Bestimmen der jeweiligen Produkteignung durchgeführt wird, wobei hierbei insbesondere ein Datenaustausch zwischen dem Mobilgerät und der externen Datenverarbeitungseinrichtung durch eine erste Kommunikationseinrichtung des Mobilgeräts und durch eine zweite Kommunikationseinrichtung der externen Datenverarbeitungseinrichtung durchgeführt wird.

Alternativ kann jedoch die Datenbank und die Steuerung insbesondere Teil des Mobilgeräts sein.

Die entsprechende durch die Steuerung bei der Bestimmung der jeweiligen Produkteignung berücksichtigte Mehrzahl von Autointerieurprodukten kann insbesondere die gesamte Vielzahl von in der Datenbank hinterlegten Autointerieurprodukten umfassen. Alternativ kann die entsprechende durch die Steuerung berücksichtigte Mehrzahl von Autointerieurprodukten nur eine Unterauswahl der Vielzahl von in der Datenbank hinterlegten Autointerieurprodukten umfassen.

Das Bestimmen der jeweiligen Produkteignung wird in Abhängigkeit von dem eingegebenen zumindest einen Körperparameter und von dem zumindest einen eingegebenen Eingabeparameter durchgeführt, so dass die bestimmte jeweilige Produkteignung die individuellen Charakteristika des Autofahrers berücksichtigt.

Beispielsweise umfasst die bestimmte jeweilige Produkteignung einen numerischen Wert, z.B. 9, oder eine Prozentangabe, z.B. 90%.

Anschließend bestimmt die Steuerung eine priorisierte Liste der Mehrzahl von Autointerieurprodukten, wobei in der priorisierten Liste die Priorität der jeweiligen Autointerieurprodukte mit zunehmender Produkteignung steigt.

Durch die priorisierte Liste wird somit ein vorteilhaftes Ranking der Mehrzahl von Autointerieurprodukten mit zunehmender Produkteignung erstellt. Umfasst die Mehrzahl von Autointerieurprodukten beispielsweise drei Autointerieurprodukte mit jeweiligen Produkteignungen von 80%, 90% und 95%, so weist das Autointerieurprodukt mit einer Produkteignung von 95% die höchste Priorität auf, weist das Autointerieurprodukt mit einer Produkteignung von 90% die zweithöchste Priorität auf, und weist das Autointerieurprodukt mit einer Produkteignung von 80% die dritthöchste Priorität auf.

Anschließend wird durch das Mobilgerät eine Auswahl von zumindest einem Autointerieurprodukt, insbesondere ein einziges Autointerieurprodukt oder mehrere Autointerieurprodukte, der priorisierten Liste der Mehrzahl von Autointerieurprodukten durch das Mobilgerät ausgegeben. Die Auswahl umfasst das Autointerieurprodukt oder die Autointerieurprodukte dessen bestimmte jeweilige Produkteignung die geringste Differenz oder gar keine Differenz zu einer maximalen Produkteignung aufweist.

Insbesondere kann die geringste Differenz der bestimmten jeweiligen Produkteignung zu einer maximalen Produkteignung Null betragen, so dass in diesem Fall gar keine Differenz zwischen der bestimmten jeweiligen Produkteignung und der maximalen Produkteignung vorhanden ist.

Insbesondere entspricht die maximale Produkteignung 100%, und es wird das zumindest eine Autointerieurprodukt ausgegeben, dessen jeweilige Produkteignung die geringste Differenz oder gar keine Differenz zu der Prozentangabe von 100% aufweist.

Insbesondere umfasst das Ausgeben das Ausgeben einer Auswahl von mehreren Autointerieurprodukten für den Autofahrer durch das Mobilgerät, wobei die Auswahl die mehreren Autointerieurprodukte der priorisierten Liste der Mehrzahl von Autointerieurprodukten umfasst, deren jeweilige Differenz zwischen der jeweiligen Produkteignung und der maximalen Produkteignung geringer als ein Schwellenwert ist. Insbesondere umfasst der Schwellenwert insbesondere einen Wert von 10%, 5 %, 2%, 1% oder beliebige andere Werte.

Somit kann aus der Vielzahl von in der Datenbank hinterlegten Autointerieurprodukten anhand der jeweiligen bestimmten Produkteignung durch das Mobilgerät eine spezifische Unterauswahl von zumindest einem Autointerieurprodukt für den Autofahrer ausgegeben werden.

Das Ausgeben der Auswahl des zumindest einen Autointerieurprodukts umfasst insbesondere das Darstellen der Auswahl auf einem Display des Mobilgeräts.

Ein Mobilgerät im Sinne der vorliegenden Anmeldung umfasst hierbei insbesondere ein tragbares elektronisches Gerät, insbesondere ein Smartphone, ein Smart-Pad, eine Smartwatch, ein Notebook oder dergleichen.

Somit kann durch das Mobilgerät in Kombination mit der Steuerung eine automatisierte und individualisierte Auswahl von zumindest einem Autointerieurprodukt für den Autofahrer bereitgestellt werden.

Aufgrund der geringen Größe des Mobilgeräts und der in das Mobilgerät integrierten Kamera kann die Aufnahme des zumindest einen Körperteils und die darauf basierende Ausgabe der Auswahl des zumindest einen Autointerieurprodukts einfach, schnell und für den Autofahrer mit einem besonders geringen Kostenaufwand, sowie durch die Berücksichtigung der Vielzahl von in der Datenbank hinterlegten Autointerieurprodukten durch die Steuerung mit einer geringen Fehleranfälligkeit erfolgen.

In einer Ausführungsform umfasst das Ausgeben das Ausgeben einer Auswahl von mehreren Autointerieurprodukten für den Autofahrer durch das Mobilgerät, wobei die Auswahl die mehreren Autointerieurprodukte der priorisierten Liste der Mehrzahl von Autointerieurprodukten umfasst, deren jeweilige Differenz zwischen der jeweiligen Produkteignung und der maximalen Produkteignung geringer als ein Schwellenwert ist, wobei der Schwellenwert insbesondere weniger als 20%, 10%, 5%, 2% oder 1% der maximalen Produkteignung beträgt.

Durch die Ausgabe von mehreren Autointerieurprodukten wird dem Autofahrer ein vorteilhafter Vergleich zwischen unterschiedlichen Autointerieurprodukten ermöglicht. Dadurch, dass nur die Autointerieurprodukte der priorisierten Liste angezeigt werden, deren jeweilige Differenz zwischen der jeweiligen Produkteignung und der maximalen Produkteignung geringer als ein Schwellenwert ist, wird sichergestellt, dass der Autofahrer nicht zu viele Autointerieurprodukte vergleichen muss, sondern sich auf die für ihn besonders passende Autointerieurprodukte fokussieren kann.

In Abhängigkeit von der Anzahl der mehreren Autointerieurprodukte in der priorisierten Liste der Mehrzahl von Autointerieurprodukten kann hierbei insbesondere der Schwellenwert angepasst werden, um dem Autofahrer eine vorteilhafte Auswahl zu ermöglichen, wobei der Schwellenwert insbesondere weniger als 20%, 10%, 5%, 2% oder 1% der maximalen Produkteignung beträgt.

In einer Ausführungsform umfasst das Verfahren das Auswählen zumindest eines Autositzes, insbesondere von zumindest einem Polsterpad eines Autositzes, aus einer Vielzahl von in einer Datenbank hinterlegten Autositzen, insbesondere von einer Vielzahl von in einer Datenbank hinterlegten Polsterpads, für einen Autofahrer, umfassend Eingeben von zumindest einem Eingabeparameter, insbesondere Autotyp, Einsatzbereich des Autos, gewünschte Härte der Sitzpolsterung, und/oder anatomische Besonderheiten des Autofahrers durch die Eingabevorrichtung des Mobilgeräts, umfassend Aufnehmen eines Fotos von zumindest einem Körperteil des Autofahrers durch die Kamera des Mobilgeräts, wobei das Bestimmen des zumindest eines Körperparameters des Autofahrers das Bestimmen einer Schulterbreite, insbes. der äußeren Schulterbreite, eines Sitzknochenabstands, einer Kniehöhe, einer Hüftbreite, einer Schrittlänge und/einer Körpergröße des Autofahrers auf Basis des aufgenommenen Fotos von zumindest einem Körperteil des Autofahrers durch die Steuerung umfasst, wobei das Bestimmen der jeweiligen Produkteignung das Bestimmen jeweils einer Sitzeignung, insbesondere Padeignung, einer Mehrzahl von Autositzen, insbesondere Polsterpads, der Vielzahl von in der Datenbank hinterlegten Autositzen, insbesondere Polsterpads, in Abhängigkeit der bestimmten Schulterbreite, insbes. der äußeren Schulterbreite, Sitzknochenabstand, Kniehöhe, Hüftbreite, Schrittlänge und/oder Körpergröße und in Abhängigkeit des zumindest einen eingegebenen Eingabeparameters durch die Steuerung umfasst, wobei das Bestimmen der priorisierten Liste das Bestimmen einer priorisierten Liste einer Mehrzahl von Autositzen, insbesondere Polsterpads, durch die Steuerung umfasst, wobei in der priorisierten Liste die Priorität der jeweiligen Autositze, insbesondere Polsterpads mit zunehmender Sitzeignung, insbesondere Padeignung, steigt, und wobei das Ausgeben der Auswahl das Ausgeben von zumindest einem Autositz, insbesondere Polsterpad, für den Autofahrer durch das Mobilgerät umfasst, wobei die Auswahl den zumindest einen Autositz, insbesondere Polsterpad, der Mehrzahl von Autositzen, insbesondere Polsterpads, umfasst, dessen jeweilige bestimmte Sitzeignung, insbesondere Padeignung, die geringste Differenz oder gar keine Differenz zu einer maximalen Sitzeignung, insbesondere Padeignung, aufweist.

Dadurch kann eine besonders vorteilhafte Ausgabe von individuell an den Autofahrer angepassten Autositzen, insbesondere Polsterpads, sichergestellt werden.

Die Polsterpads können insbesondere eine einziges Polsterpad oder eine Mehrzahl von Polsterpads umfassen.

Ein Polsterpad eines Autositzes umfasst hierbei insbesondere eine Polsterauflage, welche auf einer Sitzfläche eines Autositzes angebracht wird, um ein für den Autofahrer ergonomisch besonders optimales Sitzen auf dem Autositz zu ermöglichen.

Das durch die Kamera des Mobilgeräts aufgenommene Foto umfasst insbesondere ein Foto des gesamten Autofahrers, so dass auf Basis von diesem Foto alle relevanten Körperparameters des Autofahrers, wie Schulterbreite, insbes. der äußeren Schulterbreite, Sitzknochenabstand, Kniehöhe, Hüftbreite, Schrittlänge und/oder Körpergröße bestimmt werden kann.

Insbesondere kann das aufgenommene Foto jedoch auch zumindest ein Foto von einem Körperteil des Autofahrers bzw. mehrere Fotos von mehreren Körperteilen des Autofahrers umfassen, auf dessen bzw. auf deren Basis alle relevanten Körperparameters des Autofahrers, wie Schulterbreite, insbes. der äußeren Schulterbreite, Sitzknochenabstand, Kniehöhe, Hüftbreite, Schrittlänge und/oder Körpergröße bestimmt werden.

Um zu einer personalisierten, d.h. individualisierten Auswahl von zumindest einem Autositz, insbesondere Polsterpad des Autositzes, für den Autofahrer zu gelangen, muss die Steuerung noch über zumindest einen in der Steuerung hinterlegten Eingabeparameter, insbesondere z.B. gewünschte Härte der Sitzpolsterung, des Autofahrers verfügen.

Der zumindest eine Eingabeparameter wird hierbei direkt durch die Eingabevorrichtung, insbesondere ein Touch-Display und/oder Touch-Button des Mobilgeräts, eingegeben, um den zumindest einen Eingabeparameter der Steuerung zur Verfügung zu stellen.

In Abhängigkeit des zumindest einen Eingabeparameters und der bestimmten Schulterbreite, insbes. der äußeren Schulterbreite, Sitzknochenabstand, Kniehöhe, Hüftbreite, Schrittlänge und/oder Körpergröße bestimmt die Steuerung die jeweilige Sitzeignung, insbesondere Padeignung, insbesondere als einen numerischen Wert oder als eine Prozentangabe, wobei anschließend einer priorisierte Liste mit aufsteigender Sitzeignung, insbesondere Padeignung durch die Steuerung bestimmt wird.

Anschließend gibt das Mobilgerät zumindest einen Autositz, insbesondere Polsterpad, für den Autofahrer aus, dessen jeweilige bestimmte Sitzeignung, insbesondere Padeignung die geringste Differenz oder gar keine Differenz zu einer maximalen Sitzeignung, insbesondere Padeignung, insbesondere 100%, aufweist, insbesondere durch eine graphische Übersicht des zumindest einen ausgewählten Autositzes, insbesondere Polsterpads, insbesondere auf einem Display des Mobilgeräts.

Die geringste Differenz zwischen der jeweiligen bestimmten Sitzeignung, insbesondere Padeignung, und der maximalen Sitzeignung, insbesondere Padeignung, kann insbesondere auch Null betragen, so dass in diesem Fall gar keine Differenz vorhanden ist.

In einer Ausführungsform umfasst das Aufnehmen des Fotos das Erfassen eines Gesäßabdrucks des Autofahrers durch eine Erfassungsvorrichtung, wobei der Gesäßabdruck jeweils einen Druckbereich der beiden Sitzknochen des auf der Erfassungsvorrichtung sitzenden Autofahrers umfasst und das Aufnehmen eines Fotos des erfassten Gesäßabdrucks durch die Kamera des Mobilgeräts, und wobei das Bestimmen des zumindest einen Körperparameters das Bestimmen eines Sitzknochenabstands des Autofahrers auf Basis des aufgenommenen Fotos des erfassten Gesäßabdrucks durch die Steuerung umfasst.

Hierbei sitzt der Autofahrer zuerst auf der Erfassungsvorrichtung um den Gesäßabdruck des Autofahrers zu erhalten. Der Gesäßabdruck umfasst jeweils einen Druckbereich der beiden Sitzknochen des auf der Erfassungsvorrichtung sitzenden Autofahrers.

Das durch die Kamera des Mobilgeräts aufgenommene Foto des Gesäßabdrucks kann anschließend durch die Steuerung, insbesondere in Kombination mit einem auf dem Mobilgerät installierten Programm bzw. App, ausgewertet werden, um auf Basis des aufgenommenen Fotos des Gesäßabdrucks den Sitzknochenabstand des Autofahrers zu bestimmen.

In einer Ausführungsform umfasst die Erfassungsvorrichtung eine Grundplatte mit Noppen, auf welchen ein Blatt Papier angeordnet ist, wobei der Autofahrer während des Erfassens des Gesäßabdrucks mit seinem Gesäß auf dem Papier sitzt, und wobei während des Erfassens des Gesäßabdrucks die Noppen durch das Papier hindurchgedrückt werden, um die Druckbereiche der beiden Sitzknochen des Autofahrers auf dem Papier zu erhalten, wobei insbesondere auf dem Blatt Papier ein umlaufender Rahmen angeordnet ist, und wobei das Bestimmen des Sitzknochenabstands insbesondere das Erkennen und Rektifizieren sowie Ausrichten des aufgenommenen Fotos des Gesäßabdrucks auf Basis des umlaufenden Rahmens durch die Steuerung umfasst.

Dadurch wird der technische Vorteil erreicht, dass eine einfache, schnell durchzuführende und für den Autofahrer nicht unangenehme Erfassung des Gesäßbereichs erreicht wird. Hierbei wird insbesondere sichergestellt, dass spezifisch in dem Bereich des Papiers, auf dem der Autofahrer mit den beiden Sitzknochen sitzt, die Noppen durch das Papier hindurchgedrückt werden. Insbesondere in den anderen Bereichen des Papiers, welche nicht in Kontakt mit dem Gesäß des Autofahrers kommen, wird sichergestellt, dass die Noppen der Grundplatte nicht durch das Papier durchgedrückt werden. Diese Vorgehensweise und Verwendung ist im Detail u.a. in EP 2 508 126 A1/B1 erläutert, dargestellt und offenbart.

Wird das Papier anschließend von der Grundplatte mit den Noppen entfernt, ist der Gesäßabdruck des Autofahrers visuell auf dem Papier sichtbar und durch die Kamera des Mobilgeräts kann vorteilhaft ein Foto des Gesäßabdrucks aufgenommen und durch die Steuerung anschließend weiter ausgewertet werden.

Der auf dem Blatt Papier angeordnete umlaufende Rahmen stellt sicher, dass vor dem Auswerten des Fotos des Gesäßabdrucks das aufgenommene Foto durch die Steuerung wirksam ausgerichtet und die Größe kalibriert werden kann. Der umlaufende Rahmen dient ferner der Kalibrierung der Größenverhältnisse des aufgenommenen Fotos und insbesondere des Gesäßabdrucks. Der umlaufende Rahmen erlaubt der Steuerung insbesondere den Bereich des Papiers innerhalb des Rahmens von dem Bereich des Papiers außerhalb des Rahmens abzugrenzen, um beispielweise außerhalb des Rahmens in dem Foto angeordnete Objekte bei der Auswertung des Fotos auszuschließen. Der umlaufende Rahmen dient als Referenzhintergrund mit einer definierten Größe. Mittels des umlaufenden Rahmens kann die Steuerung den Gesäßabdruck und den ermittelten Sitzknochenabstand zu der definierten Größe des umlaufenden Rahmens in Bezug setzen, um dadurch die genaue Abmessung des Sitzknochenabstands präzise zu bestimmen.

In einer Ausführungsform umfasst die Erfassungsvorrichtung ein lichttransparentes mit Gel gefülltes Sitzkissen, wobei der Autofahrer während des Erfassens des Gesäßabdrucks mit seinem Gesäß auf dem Sitzkissen sitzt, und wobei während des Erfassens des Gesäßabdrucks sich die Verteilung des Gels innerhalb des Sitzkissens verändert, um eine visuelle Darstellung der Druckbereiche der beiden Sitzknochen des Autofahrers auf dem Sitzkissen zu erhalten. Diese Vorgehensweise und Verwendung ist im Detail u.a. in DE 20 2006 008 296 U1 erläutert, dargestellt und offenbart.

Dadurch wird der technische Vorteil erreicht, dass nach dem Aufstehen des Autofahrers von der Erfassungseinrichtung, durch die Sitzknochen des Autofahrers eine dem Gesäßabdruck des Autofahrers entsprechende und auf der Änderung der Verteilung des Gels in dem Sitzkissen basierende Vertiefung an der Oberfläche des Sitzkissens erhalten wird. Die dem Gesäßabdruck entsprechende Vertiefung an der Oberfläche des Sitzkissens kann visuell erfasst werden, und ist auch auf dem durch die Kamera des Mobilgeräts aufgenommenen Foto des Sitzkissens sichtbar und kann durch die Steuerung zur Bestimmung des Sitzknochenabstands ausgewertet werden.

In einer Ausführungsform umfasst das Bestimmen des Sitzknochenabstands des Autofahrers die folgenden Verfahrensschritte: Bestimmen eines ersten Mittelpunkts eines in dem aufgenommenen Foto des erfassten Gesäßabdrucks visuell dargestellten ersten Druckbereichs eines ersten Sitzknochens des Autofahrers durch die Steuerung, Bestimmen eines zweiten Mittelpunkts eines in dem aufgenommenen Foto des erfassten Gesäßabdrucks visuell dargestellten zweiten Druckbereichs eines zweiten Sitzknochens des Autofahrers durch die Steuerung, und Bestimmen eines Abstands zwischen dem ersten und zweiten Mittelpunkt durch die Steuerung, um den Sitzknochenabstand des Autofahrers zu bestimmen.

Dadurch wird der technische Vorteil erreicht, dass eine wirksame Bestimmung des Sitzknochenabstands des Autofahrers erreicht wird.

Insbesondere wird die Bestimmung des Sitzknochenabstands durch eine Steuerung einer externen Datenverarbeitungseinrichtung oder durch eine Steuerung des Mobilgeräts durchgeführt.

Insbesondere wird zur Auswertung des Fotos des Gesäßabdrucks das Bildauswertungsverfahren der "Blob-Detektion" verwendet. Als sog. "Blobs" werden runde Objekte bezeichnet. Blobs sind insbesondere die Durchstoßpunkte der Noppen durch das Papier. Durch den Schattenwurf erscheinen diese als dunkle runde Kreise, sogenannte Blobs. Die Blobs ergeben zusammen den Gesäßabdruck der beiden Sitzknochen. Lediglich exemplarisch wird auf die folgenden Verfahren zur "Blob-Detektion" verwiesen, https://docs.opencv.org/3.4/d0/d7a/classcv_1_1SimpleBlobDetector.html und https://ieeexplore.ieee.org/document/7467122.

Insbesondere wird hierbei ein "Gaussian Mixture Model" mit zwei Zentroiden über die Blobs bzw. die runden Objekte gelegt, und die Blobs bzw. die runden Objekte werden hierbei entsprechend ihrer Größe gewichtet. Insbesondere weisen die Zentroide jeweils einen Mittelwert und eine Varianz auf. Als Referenz wird hierbei verwiesen auf: Bishop, C., Pattern Recognition and Machine Learning (Information Science and Statistics), Springer, 1. Auflage 2007, Seite 423 ff. und http://www.cse.psu.edu/∼rtc12/CSE586Spring2010/papers/prmIMixturesEM.pdf.

Um Ausreißer zu umgehen, wird insbesondere die Auftrittswahrscheinlichkeit eines jeden runden Objekts zu den gegebenen Zentroiden berechnet. Ist diese unter einem Schwellwert, wird dieses runde Objekt durch die Steuerung nicht weiter berücksichtigt. Nach diesem Schritt werden die Zentroide insbesondere ein zweites Mal über die noch vorhandenen runden Objekte gelegt.

Der Abstand der beiden Mittelwerte der Zentroide entspricht dem Abstand zwischen dem ersten und zweiten Mittelpunkt in der Bild-Ebene in der Einheit Pixel. Da die Abmessungen des Rechtecks auf dem Bild bekannt sind, kann der Abstand in der Bild Ebene in einen realen Abstand mit der Einheit cm übersetzt werden, um den Sitzknochenabstand zu erhalten.

In einer Ausführungsform umfasst das Aufnehmen eines Fotos das Aufnehmen eines Fotos des gesamten Körpers des Autofahrers durch die Kamera des Mobilgeräts, und umfasst das Bestimmen des zumindest einen Körperparameters das Bestimmen der Körpergröße mit den folgenden Verfahrensschritten: Bestimmen eines obersten Punkts, insbesondere an dem Skalp, des Autofahrers auf dem Foto durch die Steuerung, Bestimmen eines untersten Punkts, insbesondere an dem Mittelpunkt zwischen den beiden Fersen, des Autofahrers auf dem Foto durch die Steuerung, und Bestimmen einer Differenz zwischen dem obersten Punkt und dem untersten Punkt durch die Steuerung, um die Körpergröße des Autofahrers zu bestimmen.

Dadurch wird der technische Vorteil erreicht, dass eine wirksame Erfassung der Körpergröße aus dem Foto durch die Steuerung erreicht wird.

Insbesondere kann die Steuerung auf Basis der bestimmten Körpergröße des Autofahrers weitere Körperparameter des Autofahrers, wie z.B. Schulterbreite, insbes. der äußeren Schulterbreite, Kniehöhe, Hüftbreite und/oder Schrittlänge ableiten.

In einer Ausführungsform umfasst das Aufnehmen eines Fotos das Aufnehmen eines Fotos des gesamten Körpers des Autofahrers durch die Kamera des Mobilgeräts, und umfasst das Bestimmen des zumindest einen Körperparameters das Bestimmen der Schulterbreite, insbes. der äußeren Schulterbreite, Sitzknochenabstand, Kniehöhe, Hüftbreite, und/oder Schrittlänge mit den folgenden Verfahrensschritten: Bestimmen eines obersten Punkts, insbesondere an dem Skalp, des Autofahrers auf dem Foto durch die Steuerung, Bestimmen eines untersten Punkts, insbesondere an dem Mittelpunkt zwischen den beiden Fersen, des Autofahrers auf dem Foto durch die Steuerung, Bestimmen einer Differenz zwischen dem obersten Punkt und dem untersten Punkt durch die Steuerung, um die Körpergröße des Autofahrers zu bestimmen, und Multiplizieren der bestimmten Körpergröße mit jeweils einem Faktor, wobei der jeweilige Faktor in Abhängigkeit von der Schulterbreite, insbes. der äußeren Schulterbreite, von dem Sitzknochenabstand, von der Kniehöhe, von der Hüftbreite, und/oder von der Schrittlänge festgelegt ist, um die Schulterbreite, insbes. der äußeren Schulterbreite, den Sitzknochenabstand, die Kniehöhe, die Hüftbreite, und/oder die Schrittlänge zu bestimmen.

Dadurch wird der technische Vorteil erreicht, dass eine wirksame Erfassung der Schulterbreite, insbes. der äußeren Schulterbreite, des Sitzknochenabstands, der Kniehöhe, der Hüftbreite, und/oder der Schrittlänge aus dem Foto durch die Steuerung erreicht wird.

Da insbesondere das Verhältnis zwischen Körpergröße und Schulterbreite, insbes. der äußeren Schulterbreite, Sitzknochenabstand, Kniehöhe, Hüftbreite und/oder Schrittlänge in der Regel bei unterschiedlichen Personen gleich ist, kann hierbei insbesondere für jeden spezifischen Körperparameter ein bestimmter spezifischer Faktor vorbestimmt sein, wobei der jeweilige Faktor mit dem die Körpergröße multipliziert wird, um den spezifischen Körperparameter zu erhalten.

Insbesondere wird die Körpergröße mit einem Schrittlängenfaktor zwischen 0 und 1 multipliziert wird, um die Schrittlänge zu bestimmen. Wenn der Schrittlängenfaktor beispielsweise 0,45 beträgt ergibt sich dadurch eine Schrittlänge welche 45% der Körpergröße beträgt.

Ein entsprechender Wert für einen Kniehöhenfaktor zwischen 0 und 1, mit welchem die Körpergröße multipliziert wird, um die Kniehöhe zu erhalten, kann insbesondere der Steuerung vorliegen.

Ein entsprechender Wert für einen Hüftbreitenfaktor zwischen 0 und 1, mit welchem die Körpergröße multipliziert wird, um die Hüftbreite zu erhalten, kann insbesondere der Steuerung vorliegen.

Ein entsprechender Wert für einen Schulterbreitenfaktor zwischen 0 und 1, mit welchem die Körpergröße multipliziert wird, um die Schulterbreite, insbes. der äußeren Schulterbreite, zu erhalten, kann insbesondere der Steuerung vorliegen.

Ein entsprechender Wert für einen Sitzknochenabstandsfaktor zwischen 0 und 1, mit welchem die Körpergröße multipliziert wird, um den Sitzknochenabstand zu erhalten, kann insbesondere der Steuerung vorliegen.

In einer Ausführungsform umfasst das Bestimmen des zumindest einen Körperparameters das Bestimmen der Schrittlänge mit den folgenden Verfahrensschritten: Bestimmen eines obersten Punkts, insbesondere an dem Skalp, des Autofahrers auf dem Foto durch die Steuerung, Bestimmen eines untersten Punkts, insbesondere an dem Mittelpunkt zwischen den beiden Fersen, des Autofahrers auf dem Foto durch die Steuerung, Bestimmen eines mittleren Punkts, insbesondere an der Hüfte, des Autofahrers auf dem Foto durch die Steuerung, Bestimmen des Abstands zwischen dem untersten Punkt und dem mittleren Punkt, und Reduzieren des Abstands, um die Schrittlänge des Autofahrers zu bestimmen.

Dadurch wird der technische Vorteil erreicht, dass eine wirksame Erfassung der Schrittlänge aus dem Foto durch die Steuerung erreicht wird.

Insbesondere wird das Reduzieren des Abstands dadurch erreicht, dass der Abstand zwischen dem untersten Punkt und dem mittleren Punkt mit einem Schrittlängenfaktor zwischen 0 und 1 multipliziert wird, um die Schrittlänge zu bestimmen.

Da insbesondere das Verhältnis zwischen Schrittlänge und Körpergröße bei unterschiedlichen Personen gleich ist, kann hierbei insbesondere der Schrittlängenfaktor vorbestimmt sein.

Wenn der Schrittlängenfaktor beispielsweise 0,95 beträgt reduziert die Steuerung den Abstand zwischen dem untersten Punkt und dem mittleren Punkt um ca. 5%, um die Schrittlänge des Autofahrers zu bestimmen.

In einer Ausführungsform umfasst das jeweilige Bestimmen der Produkteignung, insbesondere Sitzeignung, insbesondere Padeignung, die folgenden Verfahrensschritte: Zuordnen jeweils eines numerischen Wertes zu dem bestimmten zumindest einen Körperparameter, insbesondere zu der bestimmen Schulterbreite, insbes. der äußeren Schulterbreite, Sitzknochenabstand, Kniehöhe, Hüftbreite, Schrittlänge und/oder Körpergröße, und zu dem zumindest einen Eingabeparameter durch die Steuerung, Gewichten der jeweils zugeordneten numerischen Werte durch die Steuerung, um gewichtete numerische Werte zu erhalten, und Bilden eines Mittelwerts aus der Summe der gewichteten numerischen Werte, um die jeweilige Sitzeignung, insbesondere Padeignung zu erhalten.

Dadurch wird der technische Vorteil erreicht, dass durch die Verwendung von numerischen Werten die jeweilige Produkteignung, insbesondere Sitzeignung, insbesondere Padeignung durch die Steuerung einfach und mit einer geringen Rechenleistung ermittelt werden kann.

Insbesondere umfassen die numerischen Werte, welche zu dem bestimmten zumindest einen Körperparameter, insbesondere zu der bestimmten Schulterbreite, insbes. der äußeren Schulterbreite, Sitzknochenabstand, Kniehöhe, Hüftbreite, Schrittlänge und/oder Körpergröße und zu dem zumindest einen Eingabeparameter durch die Steuerung zugeordnet werden, numerische Werte in einem Bereich zwischen 0 und 100. Hierbei steht der untere Schwellenwert 0 des Bereichs für eine geringe Relevanz hinsichtlich der jeweiligen Produkteignung, insbesondere Sitzeignung, insbesondere Padeignung. Hierbei steht der obere Schwellenwert 100 des Bereichs für eine große Relevanz hinsichtlich der jeweiligen Produkteignung, insbesondere Sitzeignung, insbesondere Padeignung.

Das Gewichten der jeweils zugeordneten numerischen Werte durch die Steuerung berücksichtigt hierbei spezifische numerischen Werte dahingehend als besonders, dass ihnen bei der Bestimmung der jeweiligen Produkteignung, insbesondere Sitzeignung, insbesondere Padeignung eine besonders große Relevanz eingeräumt wird.

Insbesondere umfasst der zumindest eine Eingabeparameter den Autotyp, wobei insbesondere der Gewichtungsfaktor bei der Gewichtung des numerischen Wertes, welcher dem Autotyp zugeordnet ist, zwei beträgt.

Insbesondere umfasst der zumindest eine Eingabeparameter ferner den Einsatzbereich des Autos, die gewünschte Härte der Sitzpolsterung, und/oder anatomische Besonderheiten des Autofahrers, wobei insbesondere der Gewichtungsfaktor bei der Gewichtung des jeweiligen numerischen Wertes, welcher dem jeweiligen Eingabeparameter zugeordnet ist, eins beträgt.

Wenn beispielsweise der gewünschte Autotyp des Autositzes mit einem numerischen Wert von 100 hinterlegt ist, und eine Gewichtung mit dem Gewichtungsfaktor zwei stattfindet, resultiert daraus ein gewichteter numerischer Wert von 200, welcher somit die resultierende jeweilige Produkteignung, insbesondere Sitzeignung, insbesondere Padeignung, prägt.

Insbesondere kann hierbei bei der Auswahl der anatomischen Besonderheiten des Autofahrers eine mehrfache Unterauswahl getroffen werden, wenn mehrere anatomische Besonderheiten vorliegen, wobei hierbei insbesondere jede Unterauswahl bei der Gewichtung mit einem Gewichtungsfaktor von eins berücksichtigt wird.

Anschließend wird durch die Steuerung ein Mittelwert aus der Summe der gewichteten numerischen Werte gebildet, um die jeweilige Produkteignung, insbesondere Sitzeignung, insbesondere Padeignung, zu bestimmen.

In einer weiteren Ausführungsform umfasst das Verfahren das Auswählen zumindest eines Autolenkers, insbesondere von zumindest einem Griffstück eines Autolenkers, aus einer Vielzahl von in einer Datenbank hinterlegten Autolenkern, insbesondere von einer Vielzahl von in einer Datenbank hinterlegten Griffstücken, für einen Autofahrer, umfassend Eingeben von zumindest einem Eingabeparameter, insbesondere Autotyp, Einsatzbereich des Autos, und/oder anatomische Besonderheiten des Autofahrers durch eine Eingabevorrichtung eines Mobilgeräts, umfassend Aufnehmen eines Fotos von zumindest einem Körperteil des Autofahrers, insbesondere einer Hand oder des gesamten Körpers des Autofahrers, durch eine Kamera des Mobilgeräts, wobei das Bestimmen des zumindest einen Körperparameters des Autofahrers das Bestimmen einer Handlänge und/oder einer Handbreite einer Hand des Autofahrers und/oder das Bestimmen einer Körpergröße des Autofahrers auf Basis des aufgenommenen Fotos durch die Steuerung umfasst, wobei das Bestimmen der jeweiligen Produkteignung das Bestimmen jeweils einer Lenkereignung, insbesondere Griffstückeignung, einer Mehrzahl von Autolenkern, insbesondere Griffstücken, der Vielzahl von in der Datenbank hinterlegten Autolenkern, insbesondere Griffstücken, in Abhängigkeit von der bestimmten Handlänge und/oder Handbreite und/oder Körpergröße und in Abhängigkeit des zumindest einen eingegebenen Eingabeparameters durch die Steuerung umfasst, wobei das Bestimmen der priorisierten Liste das Bestimmen einer priorisierten Liste einer Mehrzahl von Autolenkern, insbesondere Griffstücken, durch die Steuerung umfasst, wobei in der priorisierten Liste die Priorität der jeweiligen Autolenker, insbesondere Griffstücke mit zunehmender Lenkereignung, insbesondere Griffstückeignung, steigt, und wobei das Ausgeben der Auswahl das Ausgeben von zumindest einem Autolenker, insbesondere Griffstück, für den Autofahrer durch das Mobilgerät umfasst, wobei die Auswahl den zumindest einen Autolenker, insbesondere Griffstück, der Mehrzahl von Autolenkern, insbesondere Griffstücken, umfasst, dessen jeweilige bestimmte Lenkereignung, insbesondere Griffstückeignung, die geringste Differenz oder gar keine Differenz zu einer maximalen Lenkereignung, insbesondere Griffstückeignung, aufweist.

Dadurch wird der technische Vorteil erreicht, dass die Kamera und die Steuerung nicht nur zur Ausgabe von zumindest einem Autositz, insbesondere Polsterpad, für den Autofahrer genutzt werden können, sondern dass auch eine Auswahl von zumindest einem Autolenker, insbesondere Griffstück für den Autofahrer durch das Mobilgerät ausgegeben werden kann.

Insbesondere umfasst das Griffstück ein einziges Griffstück oder eine Mehrzahl von Griffstücken, welcher an einem Autolenker angebracht werden, um dem Autofahrer einen besonders ergonomischen Griff des Autolenkers zu ermöglichen.

Im Vergleich zur Ausgabe des Autositzes wird bei der Ausgabe des Autolenkers, insbesondere Griffstück, jedoch insbesondere nicht die Schulterbreite, Sitzknochenabstand, Kniehöhe, Hüftbreite und/oder Schrittlänge berücksichtigt, sondern eine Handlänge und/oder eine Handbreite der Hand und/oder eine Körpergröße des Autofahrers bestimmt, und zwar durch die Aufnahme eines Fotos der Hand des Autofahrers oder des gesamten Körpers des Autofahrers durch die Kamera des Mobilgeräts und der sich anschließenden Auswertung des Fotos durch die Steuerung.

Analog zur Autositzbestimmung bzw. Polsterpad-Bestimmung wird auch bei der Autolenkerbestimmung bzw. Griffstück-Bestimmung in Abhängigkeit von der bestimmten Handlänge und/oder Handbreite und/oder Körpergröße und auf Basis des zumindest einen eingegebenen Eingabeparameters die jeweilige Lenkereignung, insbesondere Griffstückeignung, der Mehrzahl von Autolenkern, insbesondere Griffstücken, bestimmt, und anschließend durch das Mobilgerät die entsprechende Auswahl von zumindest einem Autolenker, insbesondere Griffstück, für den Autofahrer ausgegeben.

Insbesondere umfasst der zumindest eine eingegebene Eingabeparameter eine Mehrzahl von Eingabeparametern, welche insbesondere zumindest zum Teil mit den Eingabeparameter bei Bestimmung der jeweiligen Sitzeignung, insbesondere Padeignung, übereinstimmen.

In einer Ausführungsform wird das Aufnehmen des Fotos einer Hand des Autofahrers vor einem Referenzhintergrund durchgeführt, wobei der Referenzhintergrund ein Referenzsymbol mit einer definierten Größe aufweist, und wobei das Bestimmen der Handlänge und/oder der Handbreite der Hand des Autofahrers auf Basis des Fotos der Hand und auf Basis der definierten Größe des Referenzsymbols durchgeführt wird.

Dadurch wird der technische Vorteil erreicht, dass durch den Referenzhintergrund, welcher insbesondere in allen Bereichen dieselbe Farbe aufweist, ein ausreichender Kontrast in dem aufgenommenen Foto erhalten wird, welcher ausreicht, damit die Steuerung die Handlänge und/oder die Handbreite der Hand des Autofahrers auf Basis des Fotos vorteilhaft bestimmen kann.

Durch das auf dem Referenzhintergrund angeordnete Referenzsymbol mit einer definierten Größe kann die Steuerung die bestimmte Handlänge und/oder Handbreite zu der definierten Größe des Referenzsymbols in Bezug setzen, um dadurch die Abmessungen der Handlänge und/oder Handbreite präzise zu bestimmen.

In einer Ausführungsform umfasst das Aufnehmen eines Fotos das Aufnehmen eines Fotos des gesamten Körpers des Autofahrers durch die Kamera des Mobilgeräts, und umfasst das Bestimmen der Körpergröße die folgenden Verfahrensschritte: Bestimmen eines obersten Punkts, insbesondere an dem Skalp, des Autofahrers auf dem Foto durch die Steuerung, Bestimmen eines untersten Punkts, insbesondere an dem Mittelpunkt zwischen den beiden Fersen, des Autofahrers auf dem Foto durch die Steuerung, und Bestimmen einer Differenz zwischen dem obersten Punkt und dem untersten Punkt durch die Steuerung, um die Körpergröße des Autofahrers zu bestimmen.

Dadurch kann eine besonders wirksame Bestimmung der Körpergröße des Autofahrers sichergestellt werden. Hierbei wird auf die Ausführungen zur jeweiligen Sitzeignung, insbesondere Padeignung verwiesen.

In einer Ausführungsform umfasst das Bestimmen der jeweiligen Lenkereignung, insbesondere Griffstückeignung die folgenden Verfahrensschritte: Zuordnen jeweils eines numerischen Wertes zu der bestimmten Handbreite und/oder zu der bestimmten Handlänge, und/oder zu der bestimmten Körpergröße, sowie zu dem zumindest einen Eingabeparameter durch die Steuerung, Gewichten der jeweils zugeordneten numerischen Werte durch die Steuerung, um gewichtete numerische Werte zu erhalten, und Bilden eines Mittelwerts aus der Summe der gewichteten numerischen Werte, um die jeweilige Lenkereignung, insbesondere Griffstückeignung, zu erhalten.

Dadurch wird der technische Vorteil erreicht, dass eine besonders vorteilhafte Bestimmung der jeweiligen Lenkereignung, insbesondere Griffstückeignung durchgeführt werden kann. Hierbei wird auf die Ausführungen zur jeweiligen Sitzeignung, insbesondere Padeignung verwiesen.

Insbesondere umfasst der zumindest eine Eingabeparameter zur Bestimmung der jeweiligen Lenkereignung, insbesondere Griffstückeignung den Autotyp, wobei insbesondere der Gewichtungsfaktor bei der Gewichtung des numerischen Wertes, welcher dem Autotyp zugeordnet ist, zwei beträgt.

Insbesondere umfasst der zumindest eine Eingabeparameter ferner anatomische Besonderheiten des Autofahrers, wobei insbesondere der Gewichtungsfaktor bei der Gewichtung des jeweiligen numerischen Wertes, welcher dem jeweiligen Eingabeparameter zugeordnet ist, eins beträgt.

Gemäß einem zweiten Aspekt der vorliegenden Anmeldung wird die vorliegende Aufgabe durch ein System zum Auswählen zumindest eines Autointerieurprodukts aus einer Vielzahl von in einer Datenbank hinterlegten Autointerieurprodukten für einen Autofahrer gelöst, umfassend: eine Eingabevorrichtung eines Mobilgeräts, welche zum Eingeben von zumindest einem Eingabeparameter, insbesondere Autotyp, Einsatzbereich des Autos, gewünschte Härte der Sitzpolsterung, und/oder anatomische Besonderheiten des Autofahrers ausgebildet ist, eine Kamera des Mobilgeräts, welche ausgebildet ist, ein Foto von zumindest einem Körperteil des Autofahrers aufzunehmen, und eine Steuerung, welche ausgebildet ist, auf Basis des aufgenommenen Fotos zumindest einen Körperparameter des Autofahrers zu bestimmen, wobei die Steuerung ausgebildet ist, jeweils eine Produkteignung einer Mehrzahl von Autointerieurprodukten der Vielzahl von in der Datenbank hinterlegten Autointerieurprodukten in Abhängigkeit des bestimmten Körperparameters und in Abhängigkeit des zumindest einen Eingabeparameters zu bestimmen, wobei die Steuerung ausgebildet ist, eine priorisierte Liste der Mehrzahl von Autointerieurprodukten zu bestimmen, wobei in der priorisierten Liste die Priorität der jeweiligen Autointerieurprodukte mit zunehmender Produkteignung steigt, wobei das Mobilgerät ausgebildet ist, eine Auswahl von zumindest einem Autointerieurprodukt für den Autofahrer auszugeben, wobei die Auswahl das zumindest eine Autointerieurprodukt der Mehrzahl von Autointerieurprodukten umfasst, dessen jeweilige bestimmte Produkteignung die geringste Differenz oder gar keine Differenz zu einer maximalen Produkteignung aufweist.

Dadurch wird der technische Vorteil erreicht, dass durch den Nutzer des Mobilgeräts eine besonders einfache und vorteilhafte visuelle Erfassung der Auswahl des Autointerieurprodukts sichergestellt wird.

Die für das Verfahren gemäß dem ersten Aspekt genannten Ausführungsformen sind ebenso Ausführungsformen für das System gemäß dem zweiten Aspekt.

Gemäß einem dritten Aspekt wird die vorliegende Aufgabe durch ein gemäß dem ersten Aspekt ausgewähltes zumindest ein Autointerieurprodukt für den Autofahrer gelöst, welches durch ein 3D-Druckverfahren herstellbar ist.

Dadurch wird der technische Vorteil erreicht, dass ein gemäß dem ersten Aspekt individuell für den Autofahrer ausgewähltes Autointerieurprodukt, wie z.B. ein Autositz, insbesondere Polsterpad, und/oder Autolenker, insbesondere Griffstück, in einfacher und effizienter Weise herstellbar ist. Aufgrund der individuellen Anpassung des ausgewählten Autointerieurprodukts wird dieses im Gegensatz zu herkömmlichen Autointerieurprodukten nur in geringer Stückzahl hergestellt, so dass ein 3D-Druckverfahren hierbei eine kostengünstige Herstellung des Autointerieurprodukts sicherstellt. Insbesondere können die gemäß dem ersten Aspekt bereitgestellten für den Autofahrer ausgewählten Autointerieurprodukte einer entsprechenden Konfigurationsliste beim Fachverkäufer gleich hinzugefügt werden, um beim Autoverkauf Zeit und Kosten zu sparen. Insbesondere erfolgt eine individuelle Anpassung des für den Autofahrer ausgewählten Autointerieurprodukts in Bezug auf Form und/oder Härte. Hierbei wird in einer vorzugsweisen Ausführungsform eine elektronische Druckmessmatte oder -folie auf den Autositz gelegt und der Autofahrer setzt sich auf bzw. in den Autositz. Dabei werden Druckwerte erzielt und erhalten, die durch eine Software, z.B. die CAD Software der Fa. Hyperganic, ausgewertet werden kann und geänderte 3D-Modelle ausgibt. Diese werden an den 3D-Drucker übermittelt und fertigt einen Autositz oder Polsterpads nach Maß speziell für den Autofahrer.

Gemäß einem vierten Aspekt wird die vorliegende Aufgabe durch ein elektronisches Sitzeinstellungssystem eines Autos zur Autofahrer-spezifischen Einstellung eines Autositzes gelöst, wobei das Sitzeinstellungssystem zumindest einen Stellmotor aufweist, welcher ausgebildet ist, zumindest einen Bereich des Autositzes zu verfahren, und wobei der Stellmotor ausgebildet ist, den zumindest einen Bereich des Autositzes in Abhängigkeit von biometrischen Daten des Autofahrers in eine gewünschte Sitzposition zu verfahren, wobei eine Steuerung ausgebildet ist, die biometrischen Daten des Autofahrers auf Basis eines Fotos von zumindest einem Körperteil des Autofahrers zu bestimmen, wobei das Foto durch eine Kamera eines Mobilgeräts aufnehmbar ist.

Gemäß einem fünften Aspekt wird die vorliegende Aufgabe durch ein elektronisches Lenkradeinstellungssystem eines Autos zur Autofahrer-spezifischen Einstellung eines Lenkrads gelöst, wobei das Lenkradeinstellungssystem zumindest einen Stellmotor aufweist, welcher ausgebildet ist, zumindest einen Bereich des Lenkrads zu verfahren, und wobei der Stellmotor ausgebildet ist, den zumindest einen Bereich des Lenkrads in Abhängigkeit von biometrischen Daten des Autofahrers in eine gewünschte Lenkradposition zu verfahren, wobei eine Steuerung ausgebildet ist, die biometrischen Daten des Autofahrers auf Basis eines Fotos von zumindest einem Körperteil des Autofahrers zu bestimmen, wobei das Foto durch eine Kamera eines Mobilgeräts aufnehmbar ist.

Somit kann durch das Aufnehmen von zumindest einem Körperteil des Autofahrers eine einfache und vorteilhafte Bestimmung von biometrischen Daten des Autofahrers ermöglicht werden, welche wiederum für eine individuell an den Autofahrer angepasste Einstellung der gewünschten Sitzposition eines Autositzes bzw. der gewünschten Lenkradposition eines Lenkrads verwendet werden.

### Kurze Beschreibung der Figuren

Die Anmeldung wird nun unter Bezugnahme auf die beigefügten Figuren erläutert, die exemplarische und nicht beschränkende Ausführungsformen der Anmeldung zeigen, wobei
- Figur 1: ein System zum Auswählen zumindest eines Autointerieurprodukts für einen Autofahrer gemäß einer Ausführungsform in einer schematischen Darstellung,
- Figur 2: einen durch eine Erfassungsvorrichtung erfassten Gesäßabdruck eines Autofahrers gemäß einer Ausführungsform,
- Figur 3: eine Bestimmung eines Sitzknochenabstands eines Autofahrers durch eine Steuerung gemäß einer Ausführungsform,
- Figur 4: eine schematische Darstellung einer Bestimmung einer Handlänge und/oder Handbreite einer Hand eines Autofahrers für eine durch eine Steuerung bestimmte Auswahl von zumindest einem Autolenker, insbesondere Griffstück gemäß einer Ausführungsform,
- Figur 5: eine schematische Darstellung einer Bestimmung einer Schrittlänge eines Autofahrers für eine durch eine Steuerung bestimmte Auswahl von zumindest einem Autositz, insbesondere Polsterpad gemäß einer Ausführungsform,
- Figur 6: eine schematische Darstellung einer Bestimmung einer Schulterbreite, Kniehöhe, Hüftbreite, Schrittlänge und/oder Körpergröße eines Autofahrers für eine durch eine Steuerung bestimmte Auswahl von zumindest einem Autointerieurprodukt gemäß einer Ausführungsform,
- Figur 7: eine schematische Darstellung eines Verfahrens zum Auswählen zumindest eines Autointerieurprodukts für einen Autofahrer gemäß einer Ausführungsform, und
- Figur 8: eine schematische Darstellung einer Kommunikation zwischen einem Mobilgerät und einer externen Datenverarbeitungseinrichtung zum Ausführen des in Figur 7 dargestellten Verfahrens zeigt.

### Detaillierte Beschreibung der Figuren

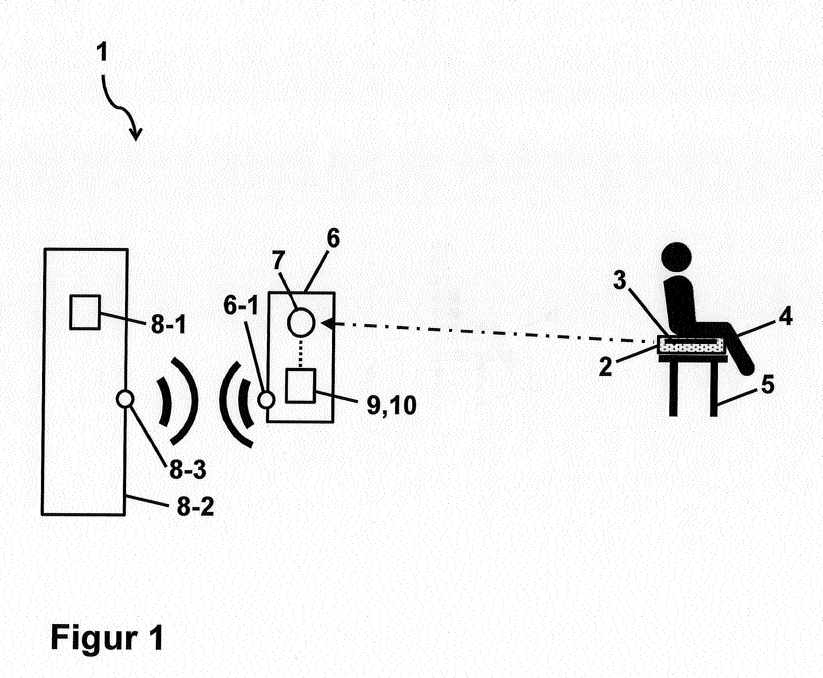

Figur 1 zeigt ein System zum Auswählen zumindest eines Autointerieurprodukts für einen Autofahrer gemäß einer Ausführungsform in einer schematischen Darstellung.

Das System 1 umfasst eine lediglich schematisch dargestellte Erfassungsvorrichtung 2, welche ausgebildet ist, einen lediglich schematisch dargestellten Gesäßabdruck 3 eines Autofahrers 4 zu erfassen.

Der in Figur 1 lediglich schematisch dargestellte Autofahrer 4 sitzt auf einer schematisch dargestellten Sitzgelegenheit 5, z.B. auf einem Hocker, wobei die Erfassungsvorrichtung 2 insbesondere zwischen der Sitzgelegenheit 5 und dem Gesäß des Autofahrer 4 angeordnet ist, um den Gesäßabdruck 3 zu erfassen.

Auch wenn dies in der Figur 1 nicht dargestellt ist, umfasst der Gesäßabdruck 3 jeweils einen Druckbereich der beiden Sitzknochen des auf der Erfassungsvorrichtung 2 sitzenden Autofahrers 4.

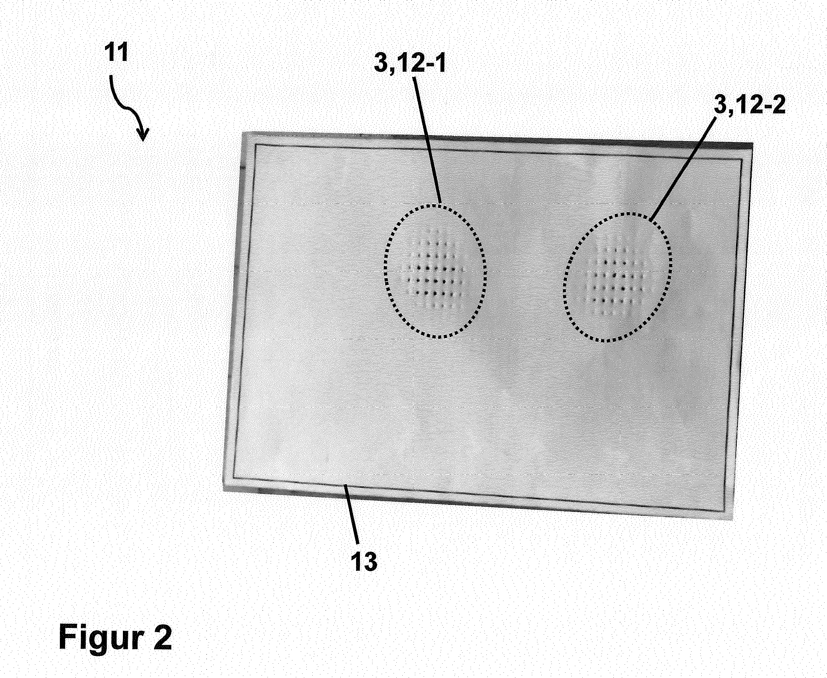

Die Erfassungsvorrichtung 2 kann insbesondere eine in Figur 1 nicht dargestellte Grundplatte mit Noppen umfassen, auf weichen ein Blatt Papier angeordnet ist, wobei der Autofahrer 4 während des Erfassens des Gesäßabdrucks 3 mit seinem Gesäß auf dem Papier sitzt, und wobei während des Erfassens des Gesäßabdrucks 3 die Noppen durch das Papier hindurchgedrückt werden, um die Druckbereiche der beiden Sitzknochen des Autofahrers 4 auf dem Papier zu erhalten. Für weitere Details zum entsprechenden Gesäßabdruck 3 wird auf die nachfolgende Figur 2 verwiesen.

Die Erfassungsvorrichtung 2 gemäß der vorliegenden Anmeldung ist jedoch nicht auf eine bestimmte Vorrichtung beschränkt, sondern kann beliebige Vorrichtungen umfassen, welche zum Erfassen eines Gesäßabdrucks 3 eines Autofahrers 4 ausgebildet sind.
Insbesondere umfasst die Erfassungsvorrichtung 2 ein lichttransparentes mit Gel gefülltes Sitzkissen, wobei der Autofahrer 4 während des Erfassens des Gesäßabdrucks 3 mit seinem Gesäß auf dem Sitzkissen sitzt, und wobei während des Erfassens des Gesäßabdrucks 3 sich die Verteilung des Gels innerhalb des Sitzkissens verändert, um eine visuelle Darstellung der Druckbereiche der beiden Sitzknochen des Autofahrers 4 auf dem Sitzkissen zu erhalten.

Das System 1 umfasst ferner ein Mobilgerät 6 mit einer Kamera 7, sowie eine in Figur 1 lediglich schematisch dargestellte Steuerung 8-1 einer externen Datenverarbeitungseinrichtung 8-2. Hierbei weist das Mobilgerät 6 insbesondere eine erste Kommunikationseinrichtung 6-1 auf, welche mit einer zweiten Kommunikationseinrichtung 8-3 der externen Datenverarbeitungseinrichtung 8-2 kommuniziert, insbesondere über eine drahtlose Kommunikationsverbindung. Die Datenverarbeitungseinrichtung 8-2 ist insbesondere als ein externer Server ausgebildet. Das Mobilgerät 6 ist insbesondere als ein Smart-Phone, als ein Smart-Pad, als eine Smart-Watch oder als ein Notebook ausgebildet. Auch wenn dies in Figur 1 lediglich schematisch dargestellt ist, ist die Kamera 7 des Mobilgeräts 6 ausgebildet, ein Foto des erfassten Gesäßabdrucks 3 des Autofahrers 4 aufzunehmen.

Offensichtlich muss der durch die Erfassungsvorrichtung 2 erfasste Gesäßabdruck 3 im Fokus der Kamera 7 des Mobilgeräts 6 positioniert werden, damit die Kamera 7 das Foto des erfassten Gesäßabdrucks 3 aufnehmen kann. Auch wenn dies in der Figur 1 nicht dargestellt ist, muss hierfür entweder die Erfassungsvorrichtung 2 zwischen dem Gesäß des Autofahrers 4 und der Sitzgelegenheit 5 entfernt werden oder der Autofahrer 4 verlässt einfach nur die Erfassungsvorrichtung 2, um den Gesäßabdruck 3 anschließend im Fokus der Kamera 7 zu positionieren und das Foto des Gesäßabdrucks 3 aufzunehmen.

Aus darstellungstechnischen Gründen ist der entsprechende Aufnahmevorgang in der Figur 1 nur schematisch durch einen Pfeil dargestellt.

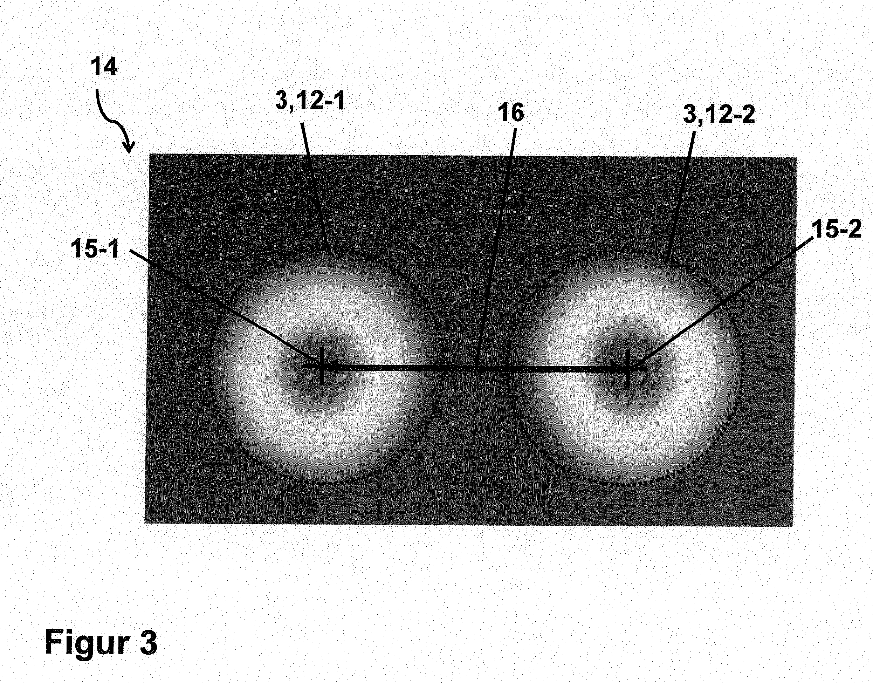

Die mit der Kamera 7 über die Kommunikationsverbindung steuerungstechnisch verbundene Steuerung 8-1 der Datenverarbeitungseinrichtung 8-2, insbesondere in Kombination mit einem auf dem Mobilgerät 6 ausgeführten Programm bzw. App, ist ausgebildet, auf Basis des aufgenommenen Fotos des Gesäßabdrucks 3 einen Sitzknochenabstand des Autofahrers 4 zu bestimmen. Für weitere Details zur Bestimmung des Sitzknochenabstands des Autofahrers 4 durch die Steuerung 8-1 wird auf die nachfolgende Figur 3 verwiesen.

Die Steuerung 8-1 ist ferner ausgebildet, jeweils eine Sitzeignung, insbesondere Padeignung einer Mehrzahl von Autositzen, insbesondere von Polsterpads, einer Vielzahl von in einer Datenbank 8-4 der externen Datenverarbeitungseinrichtung 8-2 hinterlegten Autositzen, insbesondere von Polsterpads in Abhängigkeit des bestimmten Sitzknochenabstands und in Abhängigkeit von zumindest einem Eingabeparameter, welcher durch eine Eingabevorrichtung 9 des Mobilgeräts 6 eingegeben wird, zu bestimmen. Die Eingabevorrichtung 9 umfasst insbesondere einen Touch-Display und/oder einen Touch-Button des Mobilgeräts 6.

Wurden von demselben Autofahrer 4 in der Steuerung 8-1, insbesondere durch ein auf dem Mobilgerät 6 ausgeführten Programm bzw. App, bereits Eingabeparameter hinterlegt, können diese bereits hinterlegten Eingabeparameter verwendet werden, um auf deren Basis die Bestimmung der jeweiligen Sitzeignung, insbesondere Padeignung durch die Steuerung 8-1 durchzuführen.

Der eingegebene zumindest eine Eingabeparameter ist insbesondere ausgewählt aus, Autotyp, Einsatzbereich des Autos, gewünschte Härte der Sitzpolsterung und/oder anatomische Besonderheiten des Autofahrers 4.

Die Auswahl des Autotyp kann z.B. für einen Porsche insbesondere die folgenden Unterauswahlen umfassen: 911, Cayenne, Panamera, 718, Macan, Taycan.

Die Auswahl des Einsatzbereichs des Autos kann insbesondere die folgenden Unterauswahlen umfassen: Rennstrecke, Langstrecke, Kurzstrecke.

Die Auswahl der anatomische Besonderheiten des Autofahrers 4 kann insbesondere die folgenden Unterauswahlen umfassen: Probleme mit Halswirbel, Brustwirbel, Lendenwirbel, Knie, Hüfte, Schultergelenk und/oder Steißbein bzw. Verspannungen in Schulter und/oder Nacken, ein starkes Schwitzen des Autofahrers 4, und/oder einen besonderen Komfort der Sitzfläche.

Insbesondere bestimmt die Steuerung 8-1 die jeweilige Sitzeignung, insbesondere Padeignung wie folgt:
Dem durch die Steuerung 8-1 bestimmten Sitzknochenabstand und dem zumindest einen Eingabeparameter wird durch die Steuerung 8-1 jeweils ein numerischer Wert zugeordnet. Wenn zudem durch die Steuerung 8-1 auch Schulterbreite, insbes. die äußere Schulterbreite, Kniehöhe, Hüftbreite, Schrittlänge und/oder Körpergröße des Autofahrers 4 bestimmt wurden, wird auch diesen bestimmten Werten jeweils ein numerischer Wert durch die Steuerung 8-1 zugeordnet. Für eine eventuelle Bestimmung von Schulterbreite, insbes. der äußeren Schulterbreite, Kniehöhe, Hüftbreite, Schrittlänge und/oder Körpergröße des Autofahrers 4 wird auf die nachfolgenden Ausführungen verwiesen.

Anschließend werden die jeweils zugeordneten numerischen Werte durch die Steuerung 8-1 gewichtet, um gewichtete numerische Werte zu erhalten. Die Gewichtung umfasst hierbei insbesondere jeweils einen Gewichtungsfaktor, welcher mit dem jeweiligen numerischen Wert multipliziert wird, um den jeweiligen gewichteten numerischen Wert zu erhalten.

Insbesondere beträgt der Gewichtungsfaktor bei der Gewichtung des numerischen Wertes, welcher dem Autotyp zugeordnet ist, zwei. Insbesondere beträgt der Gewichtungsfaktor bei der Gewichtung des numerischen Wertes, welcher dem Einsatzbereich des Autos, der gewünschten Härte der Sitzpolsterung und/oder anatomischen Besonderheiten des Autofahrers 4 zugeordnet ist, eins.

Anschließend wird durch die Steuerung 8-1 ein Mittelwert aus der Summe der gewichteten numerischen Werte gebildet, um die jeweilige Sitzeignung, insbesondere Padeignung zu erhalten, wobei die jeweilige Sitzeignung, insbesondere Padeignung insbesondere durch einen Prozentwert ausgegeben wird, z.B. 96%.

Anschließend bestimmt die Steuerung 8-1 eine priorisierte Liste der Mehrzahl von Autositzen, insbesondere Polsterpads, wobei in der priorisierten Liste die Priorität der jeweiligen Autositze, insbesondere Polsterpads mit zunehmender Sitzeignung, insbesondere Padeignung steigt.

Das Mobilgerät 6, insbesondere eine in Figur 1 lediglich schematisch dargestellte Ausgabevorrichtung 10 des Mobilgeräts 6, wie z.B. das Display, ist ausgebildet, eine Auswahl von zumindest einem Autositz, insbesondere Polsterpad der priorisierten Liste der Mehrzahl von Autositzen, insbesondere Polsterpads für den Autofahrer 4 auszugeben.

Das in der Figur 1 lediglich schematisch dargestellte Touch-Display des Mobilgeräts 6 ist somit als eine Eingabevorrichtung 9 und als eine Ausgabevorrichtung 10 ausgebildet. Alternativ können jedoch die Eingabevorrichtung 9 und die Ausgabevorrichtung 10 auch unterschiedliche Vorrichtungen des Mobilgeräts 6 umfassen.

Die Steuerung 8-1 legt die Auswahl des zumindest einen Autositzes, insbesondere Polsterpads, derart fest, dass die Auswahl den zumindest einen Autositz, insbesondere Polsterpad, der priorisierten Liste der Mehrzahl von Autositzen, insbesondere Polsterpads, umfasst, dessen jeweilige bestimmte Sitzeignung, insbesondere Padeignung, die geringste Differenz oder gar keine Differenz zu einer maximalen Sitzeignung, insbesondere Padeignung, wie z.B. 100 %, aufweist.

Hierbei umfassen die durch die Steuerung 8-1 bestimmten jeweiligen Sitzeignungen, insbesondere Padeignungen insbesondere numerische Werte bzw. Prozentwerte. Somit kann durch die Steuerung 8-1 bestimmt werden, welche numerischen Werte bzw. Prozentwerte der jeweiligen Sitzeignung, insbesondere Padeignung der jeweiligen Autositzen, insbesondere Polsterpads der maximalen Sitzeignung, insbesondere Padeignung, insbesondere 100 %, am nächsten kommen, um die Auswahl von zumindest einem Autositzen, insbesondere Polsterpads für den Autofahrer 4 auszugeben. Insbesondere kann ein Schwellenwert für die Differenz zwischen jeweiliger Sitzeignung, insbesondere Padeignung, und maximaler Sitzeignung, insbesondere Padeignung, durch die Steuerung 8-1 bestimmt sein. Beträgt der Schwellenwert insbesondere 5%, so werden beispielsweise nur Autositze, insbesondere Polsterpads, durch das Mobilgerät 6 dargestellt, deren jeweilige Sitzeignung, insbesondere Padeignung, zwischen 95% und 100% betragen.

Die durch das Mobilgerät 6 für den Autofahrer 4 ausgegebene Auswahl von zumindest einem Autositz, insbesondere Polsterpad, stellt dem Autofahrer 4 somit eine für ihn individualisierte Ausgabe von Autositzen, insbesondere Polsterpads, zur Verfügung, welche für den Autofahrer 4 besonders geeignet sind. Die individualisierte Auswahl, ist insbesondere an die Gesäßform des Autofahrer 4, und/oder an weitere Körperparameter des Autofahrer 4 und/oder an die Fahrgewohnheiten des Autofahrer 4 individuell angepasst.

Figur 2 zeigt einen durch eine Erfassungsvorrichtung erfassten Gesäßabdruck eines Autofahrers.

Der in Figur 2 dargestellte Gesäßabdruck 3 wird durch eine Erfassungsvorrichtung 2 erhalten, welche eine in Figur 2 nicht dargestellte Grundplatte mit Noppen umfasst, auf welcher das in Figur 2 dargestellte Blatt Papier 11 angeordnet ist. Während des Erfassens des Gesäßabdrucks 3 saß der Autofahrers 4 mit seinem Gesäß auf dem Papier 11, so dass die Noppen durch das Papier 11 hindurchgedrückt wurden, um die jeweiligen Druckbereiche 12-1, 12-2 der beiden Sitzknochen des Autofahrers 4 auf dem Papier 11 zu erhalten.

Beispielsweise resultiert der erste Druckbereich 12-1 des Gesäßabdrucks 3 aus dem linken Sitzknochen des Autofahrers 4 und resultiert der zweite Druckbereich 12-2 des Gesäßabdrucks 3 aus dem rechten Sitzknochen des Autofahrers 4.

Auf dem Blatt Papier 11 ist insbesondere ein umlaufender Rahmen 13 angeordnet. Das durch die Steuerung 8-1 durchgeführte Bestimmen eines Sitzknochenabstands umfasst hierbei das Erkennen und Rektifizieren sowie Ausrichten des aufgenommenen Fotos des Gesäßabdrucks 3 auf Basis des umlaufenden Rahmens 13 durch die Steuerung 8-1.

Für weitere Details zum Bestimmen des Sitzknochenabstands durch die Steuerung 8-1 wird auf die nachfolgende Figur 3 verwiesen.

Figur 3 zeigt eine Bestimmung eines Sitzknochenabstands eines Autofahrers durch eine Steuerung gemäß einer Ausführungsform.

In der Figur 3 ist ein durch eine Kamera 7 des Mobilgeräts 6 aufgenommenes Foto 14 eines Gesäßabdrucks 3 des Autofahrers 4 gezeigt, wobei der Gesäßabdruck 3 einen ersten Druckbereich 12-1 und einen zweiten Druckbereich 12-2 umfasst.

Die Steuerung 8-1, insbesondere im Zusammenspiel mit einem auf dem Mobilgerät 6 ausgeführten Programm bzw. App, bestimmt einen ersten Mittelpunkt 15-1 des in dem aufgenommenen Foto 14 des erfassten Gesäßabdrucks 3 visuell dargestellten ersten Druckbereichs 12-1 eines ersten Sitzknochens des Autofahrers 4.

Die Steuerung 8-1, insbesondere im Zusammenspiel mit einem auf dem Mobilgerät 6 ausgeführten Programm bzw. App, bestimmt einen zweiten Mittelpunkt 15-2 des in dem aufgenommenen Foto 14 des erfassten Gesäßabdrucks 3 visuell dargestellten zweiten Druckbereichs 12-2 eines zweiten Sitzknochens des Autofahrers 4.

Anschließend bestimmt die Steuerung 8-1 einen Abstand zwischen dem ersten und zweiten Mittelpunkt 15-1, 15-2, um den Sitzknochenabstand 16 des Autofahrers 4 zu bestimmen. Der Abstand zwischen dem ersten und zweiten Mittelpunkt 15-1, 15-2 entspricht hierbei dem Sitzknochenabstand 16 des Autofahrers 4.

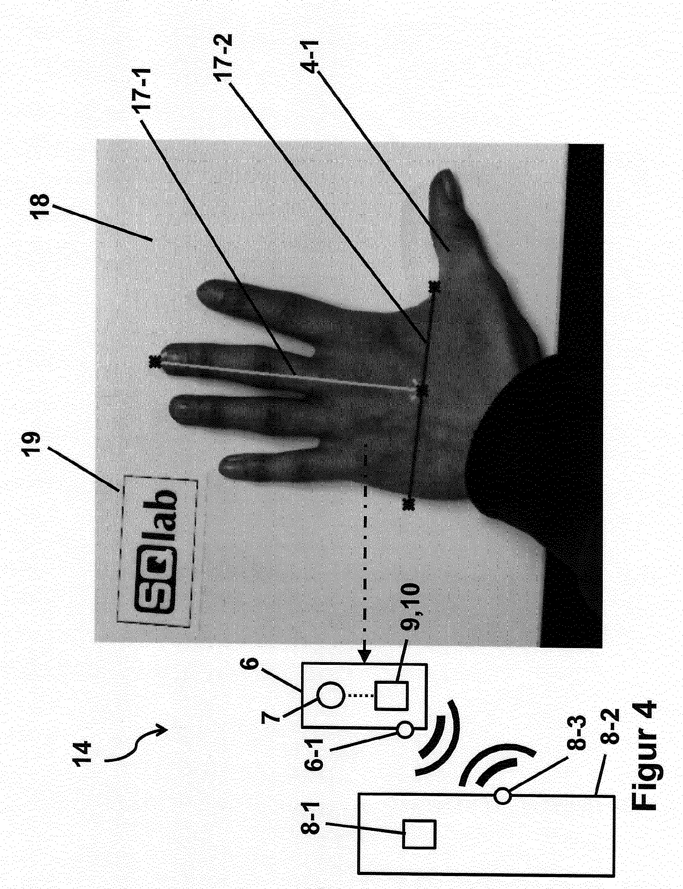

Figur 4 zeigt eine schematische Darstellung einer Bestimmung einer Handlänge und/oder Handbreite einer Hand eines Autofahrers für eine durch eine Steuerung bestimmte Auswahl von zumindest einem Autolenker, insbesondere Griffstück gemäß einer Ausführungsform.

Die in Figur 4 lediglich schematisch dargestellte Steuerung 8-1 ist ferner insbesondere zum Auswählen von zumindest einem Autolenker, insbesondere Griffstück für den Autofahrer 4 ausgebildet.

Hierzu nimmt die Kamera 7 des Mobilgeräts 6 ein Foto 14 einer Hand 4-1 des Autofahrers 4 auf. Hierbei legt der Autofahrers 4, wie in Figur 4 schematisch dargestellt ist, seine Hand 4-1 insbesondere auf einen Referenzhintergrund 18, welche insbesondere eine helle Oberfläche umfasst. Wie aus der Figur 4 hervorgeht, ist auf dem Referenzhintergrund 18 ein Referenzsymbol 19 mit einer definierten Größe angeordnet. Durch dieses angeordnete Referenzsymbol 19 mit einer definierten Größe kann die Steuerung 8-1 die bestimmte Handlänge 17-1 und die bestimmte Handbreite 17-2 zu der definierten Größe des Referenzsymbols 19 in Bezug setzen, um dadurch die Abmessungen der Handlänge 17-1 und/oder Handbreite 17-2 präzise zu bestimmen.

Wie in der Figur 4 lediglich schematisch dargestellt ist, bestimmt die Steuerung 8-1 eine Handlänge 17-1 und/oder eine Handbreite 17-2 der Hand 4-1 des Autofahrers 4 auf Basis des Fotos 14 der Hand 4-1. Insbesondere bestimmt die Steuerung 8-1 die Handlänge 17-1 und/oder die Handbreite 17-2 der Hand 4-1 des Autofahrers 4 auf Basis des Fotos 14 der Hand 4-1 und auf Basis der definierten Größe des Referenzsymbols 19.

Das Bestimmen der Handlänge 17-1 und/oder der Handbreite 17-2 der Hand 4-1 des Autofahrers 4 umfasst insbesondere die folgenden Schritte, welche insbesondere unter Verwendung des Programms "Mediapipe" durchgeführt werden (https://www.mediapipe.dev/).

In einem ersten Schritt wird durch die Steuerung 8-1 der Verlauf des Skeletts der Hand 4-1 des Autofahrers 4 erkannt.

In einem zweiten Schritt wird durch die Steuerung 8-1 die Handlänge eine erste Gerade bestimmt, die durch den Mittelfinger verläuft, wobei die erste Gerade an der Spitze des Mittelfingers beginnt und auf der Höhe des Daumens endet. Dabei wird der Beginn des Daumens senkrecht auf die erste Gerade projiziert. Um die erste Gerade zu berechnen, werden die Punkte des Mittelfingers linear interpoliert.

Um auf Basis der ersten Gerade die Spitze des Mittelfingers und den Daumen zu finden, wird die Hand 4-1 zuerst segmentiert. Hierbei wird auf der Höhe der Fingerknöchel eine in Figur 4 nicht dargestellte zweite Gerade konstruiert, die senkrecht zur ersten Gerade verläuft.

Entlang dieser zweiten Gerade wird nach den zwei Rändern der Segmentierung gesucht. Wenn die Schnittpunkte mit dem Rand der Hand 4-1 gefunden sind wird deren Abstand bestimmt. Danach wird die zweite Gerade iterativ zum Handgelenk bewegt. Bei jeder Iteration wird die Distanz der zweiten Gerade geprüft.

Wenn eine geprüfte Distanz der zweiten Gerade mehr als 10% größer ist als die vorherig geprüfte Distanz der zweiten Gerade ist, dann wurde der Daumen gefunden. Die vorherig geprüfte Distanz der zweiten Gerade entspricht der in Figur 4 dargestellten Handbreite 17-2 der Hand 4-1.

Um die Fingerspitze des Mittelfingers zu finden, wird entlang der ersten Gerade nach einem Schnittpunkt mit dem Rand der Hand 4-1 gesucht, um die Handlänge 17-1 zu erhalten.

Die Steuerung 8-1 bestimmt eine jeweilige Lenkereignung, insbesondere Griffstückeignung, einer Mehrzahl von in einer Datenbank 8-3 einer externen Datenverarbeitungseinrichtung 8-2 hinterlegten Autolenkern, insbesondere Griffstücken, in Abhängigkeit von der bestimmen Handlänge 17-1 und/oder der bestimmten Handbreite 17-2, sowie in Abhängigkeit von zumindest einem in der Steuerung 8-1 hinterlegten Eingabeparameter.

Das Bestimmen der jeweiligen Lenkereignung, insbesondere Griffstückeignung, durch die Steuerung 8-1 erfolgt analog zum Bestimmen der jeweiligen Sitzeignung, insbesondere Padeignung, wobei jedoch gegebenenfalls unterschiedliche Parameter bzw. Gewichtungen herangezogen werden.

Der zumindest eine eingegebene Eingabeparameter ist insbesondere ausgewählt aus, Autotyp, Einsatzbereich des Autos, und/oder anatomische Besonderheiten des Autofahrers 4.

Insbesondere umfasst das Bestimmen der jeweiligen Lenkereignung, insbesondere Griffstückeignung die folgenden Schritte:
Die Steuerung 8-1 ordnet jeweils einen numerischen Wert zu der bestimmten Handbreite 17-2 und/oder zu der bestimmten Handlänge 17-1 und zu dem zumindest einen Eingabeparameter zu.

Die Steuerung 8-1 gewichtet anschließend die jeweils zugeordneten numerischen Werte, um gewichtete numerische Werte zu erhalten.

Die Steuerung 8-1 bildet anschließend einen Mittelwert aus der Summe der gewichteten numerischen Werte, um die jeweilige Lenkereignung, insbesondere Griffstückeignung zu erhalten.

Insbesondere umfasst der zumindest eine Eingabeparameter den Autotyp, wobei insbesondere der Gewichtungsfaktor bei der Gewichtung des numerischen Wertes, welcher dem Autotyp zugeordnet ist, zwei beträgt.

Die Steuerung 8-1 gibt anschließend eine Auswahl von zumindest einem Autolenker, insbesondere Griffstück für den Autofahrer 4 aus, deren jeweilige Lenkereignung, insbesondere Griffstückeignung zu einer maximalen Lenkereignung, insbesondere Griffstückeignung, insbesondere 100%, die geringste Differenz oder gar keine Differenz aufweist.

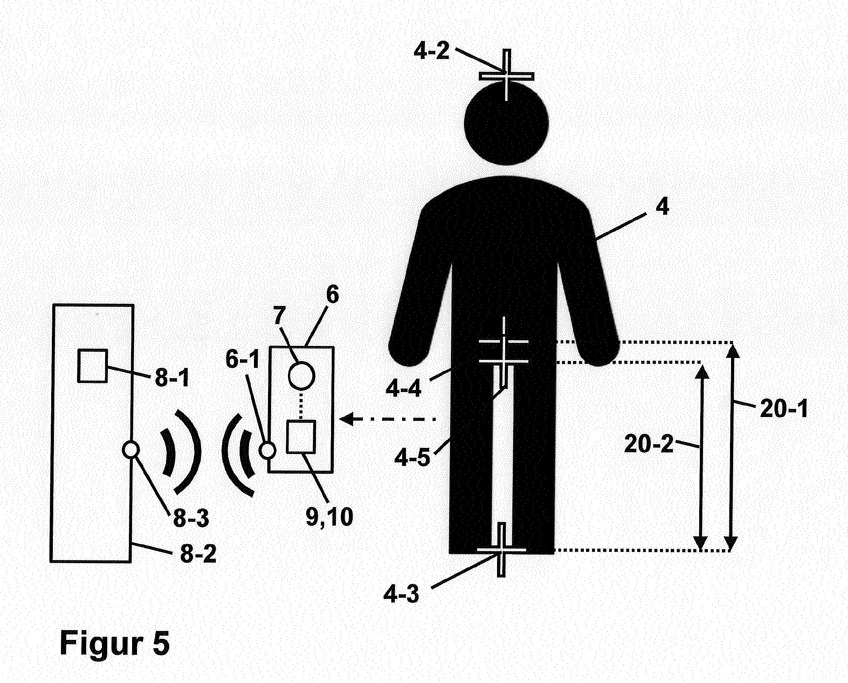

Figur 5 zeigt eine schematische Darstellung einer Bestimmung einer Schrittlänge eines Autofahrers für eine durch eine Steuerung bestimmte Auswahl von zumindest einem Autositz, insbesondere Polsterpad gemäß einer Ausführungsform.

Hierzu nimmt die Kamera 7 des Mobilgeräts 6 ein Foto 14 des gesamten Autofahrers 4 auf, und die Steuerung 8-1 bestimmt eine Schrittlänge des Autofahrers 4 auf Basis des Fotos 14 des gesamten Autofahrers 4, insbesondere unter Verwendung des Programms "Mediapipe" (https://www.mediapipe.dev/).

Beim Aufnehmen 30-2 des Fotos 14 des Autofahrers 4 ist es insbesondere von Vorteil, wenn der Autofahrer 4 von vorne fotografiert wird, wenn der Autofahrer 4 einen Großteil des Fotos 14 einnimmt, wenn alle Körperteile des Autofahrers 4 sichtbar sind, wenn keine weiteren Personen auf dem Foto 14 abgebildet sind, wenn die Füße des Autofahrers 4 in einer V-Ausrichtung stehen und die Fersen gut erkennbar sind, und/oder wenn der Aufnahmewinkel ca. 90° beträgt.

Wie in der Figur 5 schematisch gezeigt ist, bestimmt die Steuerung 8-1 auf dem aufgenommenen Foto 14 einen obersten Punkt 4-2, insbesondere an dem Skalp, des Autofahrers 4, und bestimmt die Steuerung 8-1 auf dem aufgenommenen Foto 14 einen untersten Punkt 4-3, insbesondere an dem Mittelpunkt zwischen den beiden Fersen, des Autofahrers 4.

Anschließend bestimmt die Steuerung 8-1 auf dem aufgenommenen Foto 14 einen mittleren Punkt 4-4, insbesondere an der Hüfte, des Autofahrers 4.

Anschließend bestimmt die Steuerung 8-1 auf dem aufgenommenen Foto 14 den Abstand 20-1 zwischen dem untersten Punkt 4-3 und dem mittleren Punkt 4-4. Die Steuerung 8-1 reduziert anschließend den Abstand 20-1 um ca. 5%, um die Schrittlänge 20-2 des Autofahrers 4 zwischen dem untersten Punkt 4-3 und einem Schrittpunkt 4-5 des Autofahrers 4 zu bestimmen.

Die Steuerung 8-1 bestimmt die jeweilige Sitzeignung, insbesondere Padeignung in Abhängigkeit von der bestimmten Schrittlänge 20-2, sowie in Abhängigkeit von zumindest einem eingegebenen Eingabeparameter.

Nach dem Bestimmen der priorisierten Liste durch die Steuerung 8-1 gibt das Mobilgerät 6 anschließend eine Auswahl von zumindest einem Autositz, insbesondere Polsterpad für den Autofahrer 4 aus, wobei die Auswahl den zumindest einen Autositz, insbesondere Polsterpad der Mehrzahl von Autositzen, insbesondere Polsterpads umfasst, dessen jeweilige bestimmte Sitzeignung, insbesondere Padeignung die geringste Differenz oder gar keine Differenz zu einer maximalen Sitzeignung, insbesondere Padeignung aufweist.

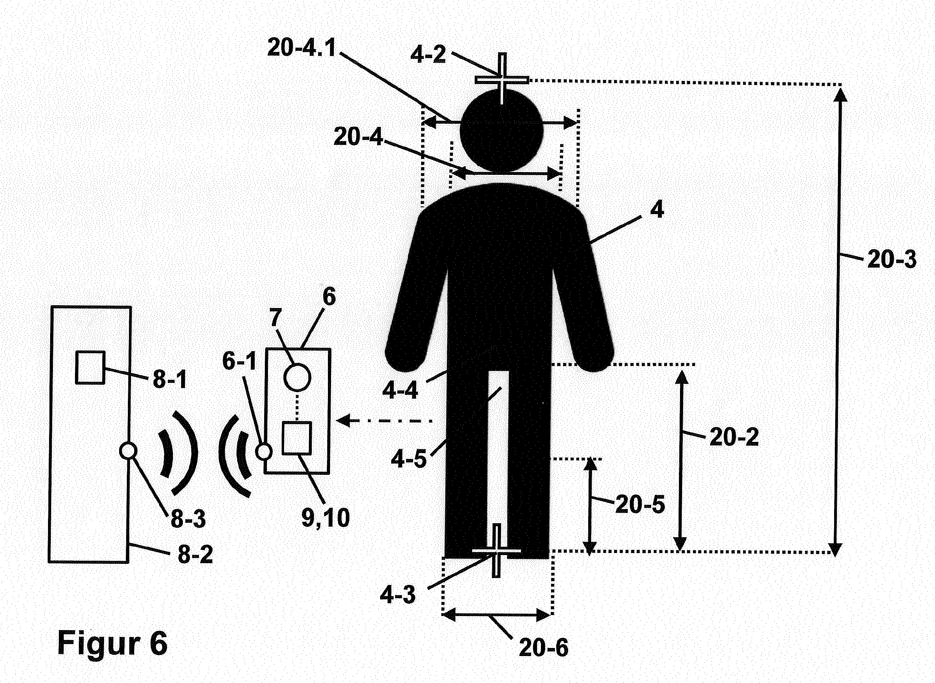

Figur 6 zeigt eine schematische Darstellung einer Bestimmung einer Schulterbreite, Kniehöhe, Hüftbreite, Schrittlänge und/oder Körpergröße eines Autofahrers für eine durch eine Steuerung bestimmte Auswahl von zumindest einem Autointerieurprodukt gemäß einer Ausführungsform.

Für Details zur Aufnahme des Fotos 14 des Autofahrers 4 wird auf die Ausführungen zur Figur 5 verwiesen.

Wie in der Figur 6 schematisch gezeigt ist, bestimmt die Steuerung 8-1 auf dem aufgenommenen Foto 14 einen obersten Punkt 4-2, insbesondere an dem Skalp, des Autofahrers 4, und bestimmt die Steuerung 8-1 auf dem aufgenommenen Foto 14 einen untersten Punkt 4-3, insbesondere an dem Mittelpunkt zwischen den beiden Fersen, des Autofahrers 4, wobei die Steuerung 8-1 die Differenz zwischen dem obersten und untersten Punkt 4-2, 4-3 als Körpergröße 20-3 des Autofahrers 4 bestimmt.

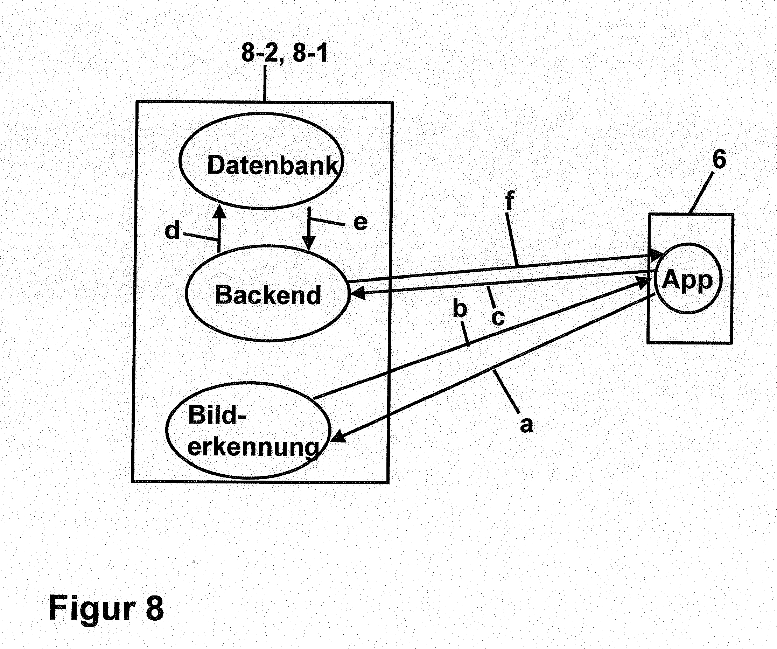

Aufgrund von bei unterschiedlichen Person oftmals annähernd gleichen Verhältnis zwischen der Körpergröße 20-3 und der Schulterbreite von Gelenk zu Gelenk 20-4, der äußeren Schulterbreite 20-4.1, Kniehöhe 20-5, Hüftbreite 20-6 bzw. Schrittlänge 20-2 kann die Körpergröße 20-3 insbesondere mit einem Faktor zwischen 0 und 1 multipliziert werden, wobei der jeweilige Faktor in Abhängigkeit von der Schulterbreite von Gelenk zu Gelenk 20-4, Kniehöhe 20-5, Hüftbreite 20-6 bzw. Schrittlänge 20-2 festgelegt ist, um die in Figur 8 lediglich schematisch dargestellte Schulterbreite von Gelenk zu Gelenk 20-4, die äußere Schulterbreite 20-4.1, Kniehöhe 20-5, Hüftbreite 20-6 bzw. Schrittlänge 20-2 zu bestimmen.

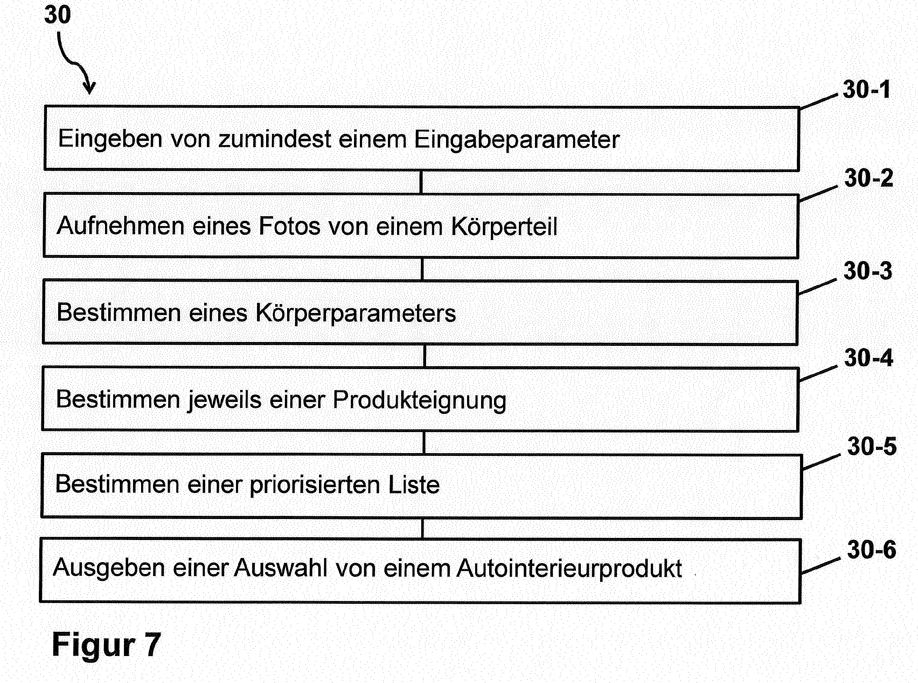

Figur 7 zeigt eine schematische Darstellung eines Verfahrens zum Auswählen zumindest eines Autointerieurprodukts für einen Autofahrer gemäß einer Ausführungsform.

Das Verfahren 30 umfasst als ersten Verfahrensschritt das Eingeben 30-1 von zumindest einem Eingabeparameter, insbesondere Autotyp, Einsatzbereich des Autos, gewünschte Härte der Sitzpolsterung, und/oder anatomische Besonderheiten des Autofahrers 4 durch eine Eingabevorrichtung 9 eines Mobilgeräts 6.

Das Verfahren 30 umfasst als zweiten Verfahrensschritt das Aufnehmen 30-2 eines Fotos 14 von zumindest einem Körperteil des Autofahrers 4 durch eine Kamera 7 des Mobilgeräts 6.

Das Verfahren 30 umfasst als dritten Verfahrensschritt das Bestimmen 30-3 zumindest eines Körperparameters des Autofahrers 4 auf Basis des aufgenommenen Fotos 14 durch eine Steuerung 8-1.

Das Verfahren 30 umfasst als vierten Verfahrensschritt das Bestimmen 30-4 jeweils einer Produkteignung einer Mehrzahl von Autointerieurprodukten der Vielzahl von in der Datenbank hinterlegten Autointerieurprodukten in Abhängigkeit des bestimmten Körperparameters und in Abhängigkeit des zumindest einen eingegebenen Eingabeparameters durch die Steuerung 8-1.

Das Verfahren 30 umfasst als fünften Verfahrensschritt das Bestimmen 30-5 einer priorisierten Liste der Mehrzahl von Autointerieurprodukten durch die Steuerung 8-1, wobei in der priorisierten Liste die Priorität der jeweiligen Autointerieurprodukte mit zunehmender Produkteignung steigt. Das Bestimmen 30-5 einer priorisierten Liste der Mehrzahl von Autointerieurprodukten umfasst auch das Erstellen eben dieser Liste.

Das Verfahren 30 umfasst als sechsten Verfahrensschritt das Ausgeben 30-6 einer Auswahl von zumindest einem Autointerieurprodukt für den Autofahrer 4 durch das Mobilgerät 6, wobei die Auswahl das zumindest eine Autointerieurprodukt der priorisierten Liste der Mehrzahl von Autointerieurprodukten umfasst, dessen jeweilige bestimmte Produkteignung eine geringste Differenz oder gar keine Differenz zu einer maximalen Produkteignung aufweist.

Figur 8 zeigt eine schematische Darstellung einer Kommunikation zwischen einem Mobilgerät und einer externen Datenverarbeitungseinrichtung zum Ausführen des in Figur 7 dargestellten Verfahrens.

In der Figur 8 ist das Mobilgerät 6 mit der darauf ausgeführten App und die externe Datenverarbeitungseinrichtung 8-2 umfassend eine Bilderkennung, ein Backend, und eine Datenbank als einzelne Untermodule der Steuerung 8-1 der Datenverarbeitungseinrichtung 8-2 lediglich schematisch dargestellt.

Nach dem Eingeben 30-1 des zumindest einen Eingabeparameters in das Mobilgerät 6 und dem Aufnehmen 30-2 des Fotos 14 von zumindest einem Körperteil des Autofahrers 4 durch eine Kamera 7 des Mobilgeräts 6, wird das aufgenommene Foto 14 von der App des Mobilgeräts 6 an das Bilderkennungsuntermodul der Steuerung 8-1 der Datenverarbeitungseinrichtung 8-2 übermittelt (dargestellt als Schritt a in Figur 8).

Die Bilderkennung als Untermodul der Steuerung 8-1 bestimmt auf Basis des aufgenommenen Fotos 14 den zumindest einen Körperparameter des Autofahrers 4 und übermittelt den zumindest einen Körperparameter zurück an die App des Mobilgeräts 6 (dargestellt als Schritt b in Figur 8). Somit verfügt die App über den zumindest einen Körperparameter.

Anschließend übermittelt die App des Mobilgeräts 6 den zumindest einen Eingabeparameter und den zumindest einen Körperparameter an das Backenduntermodul der Steuerung 8-1 der Datenverarbeitungseinrichtung 8-2 (dargestellt als Schritt c in Figur 8), wobei das Backend als Schnittstelle zur Weiterübermittlung des zumindest einen Eingabeparameters und des zumindest einen Körperparameters an das Datenbankuntermodul der Steuerung 8-1 dient (dargestellt als Schritt d in Figur 8).

Das Datenbankuntermodul der Steuerung 8-1 steht mit der in Figur 8 nicht dargestellten Datenbank der Datenverarbeitungseinrichtung 8-2 in Verbindung. Das Datenbankmodul bestimmt zuerst in Abhängigkeit von dem zumindest einen Eingabeparameter und dem zumindest einen Körperparameter jeweils eine Produkteignung einer Mehrzahl von Autointerieurprodukten einer Vielzahl von in der Datenbank hinterlegten Autointerieurprodukten, bestimmt anschließend eine in Abhängigkeit der Produkteignung priorisierte Liste der Mehrzahl von Autointerieurprodukten, und übermittelt die priorisierte Liste an das Backend zurück (dargestellt als Schritt e in Figur 8), wobei das Backend die priorisierte Liste weiter an die App des Mobilgeräts 6 zurückübermittelt (dargestellt als Schritt f in Figur 8).

Anschließend führt das Mobilgerät 6 den Verfahrensschritt Ausgeben 30-6 einer Auswahl von zumindest einem Autointerieurprodukt für den Autofahrer 4 durch, wobei die Auswahl das zumindest eine Autointerieurprodukt umfasst, dessen jeweilige bestimmte Produkteignung eine geringste Differenz oder gar keine Differenz zu einer maximalen Produkteignung aufweist.

### Bezugszeichen

- 1: System zum Auswählen zumindest eines Autointerieurprodukts
- 2: Erfassungsvorrichtung
- 3: Gesäßabdruck
- 4: Autofahrer
- 4-1: Hand
- 4-2: Oberster Punkt des Autofahrers (Kopf)
- 4-3: Unterster Punkt des Autofahrers (Füße)
- 4-4: Mittlerer Punkt des Autofahrers (Hüfte)
- 4-5: Schrittpunkt des Autofahrers
- 5: Sitzgelegenheit
- 6: Mobilgerät
- 6-1: Erste Kommunikationseinrichtung
- 7: Kamera des Mobilgeräts
- 8-1: Steuerung
- 8-2: Externe Datenverarbeitungseinrichtung
- 8-3: Zweite Kommunikationseinrichtung
- 9: Eingabevorrichtung des Mobilgeräts
- 10: Ausgabevorrichtung des Mobilgeräts
- 11: Papier
- 12-1: Erster Druckbereich
- 12-2: Zweiter Druckbereich
- 13: Umlaufender Rahmen
- 14: Foto
- 15-1: Erster Mittelpunkt
- 15-2: Zweiter Mittelpunkt
- 16: Sitzknochenabstand
- 17-1: Handlänge
- 17-2: Handbreite
- 18: Referenzhintergrund
- 19: Referenzsymbol
- 20-1: Abstand zwischen untersten Punkt 4-3 und mittlerem Punkt 4-4
- 20-2: Schrittlänge
- 20-3: Körpergröße
- 20-4: Schulterbreite von Gelenk zu Gelenk
- 20-4.1: Äußere Schulterbreite
- 20-5: Kniehöhe
- 20-6: Hüftbreite
- 30: Verfahren zum Auswählen zumindest eines Autointerieurprodukts
- 30-1: Erster Verfahrensschritt: Eingeben von zumindest einem Eingabeparameter
- 30-2: Zweiter Verfahrensschritt: Aufnehmen eines Fotos von zumindest einem Körperteil des Autofahrers
- 30-3: Dritter Verfahrensschritt: Bestimmen zumindest eines Körperparameters des Autofahrers
- 30-4: Vierter Verfahrensschritt: Bestimmen jeweils einer Produkteignung einer Mehrzahl von Autointerieurprodukten
- 30-5: Fünfter Verfahrensschritt: Bestimmen einer priorisierten Liste der Mehrzahl von Autointerieurprodukts
- 30-6: Sechster Verfahrensschritt: Ausgeben einer Auswahl von zumindest einem Autointerieurprodukt

- a: Übermitteln eines Fotos von einer App eines Mobilgeräts an eine Steuerung einer externen Datenverarbeitungseinrichtung
- b: Übermitteln zumindest eines Körperparameters von einer Steuerung einer externen Datenverarbeitungseinrichtung an eine App eines Mobilgeräts
- c: Übermitteln von zumindest einem Eingabeparameter und zumindest einem Körperparameter von einer App eines Mobilgeräts an ein Backenduntermodul einer Steuerung einer externen Datenverarbeitungseinrichtung
- d: Übermitteln von zumindest einem Eingabeparameter und zumindest einem Körperparameter von einem Backenduntermodul einer Steuerung einer externen Datenverarbeitungseinrichtung zu einem Datenbankuntermodul einer Steuerung einer externen Datenverarbeitungseinrichtung
- e: Übermitteln von einer priorisierten Liste der Mehrzahl von Autointerieurprodukten von einem Datenbankuntermodul einer Steuerung einer externen Datenverarbeitungseinrichtung zu einem Backenduntermodul einer Steuerung einer externen Datenverarbeitungseinrichtung
- f: Übermitteln von einer priorisierten Liste der Mehrzahl von Autointerieurprodukten von einem Backenduntermodul einer Steuerung einer externen Datenverarbeitungseinrichtung zu einer App eines Mobilgeräts

## Patentansprüche

1. Verfahren (30) zum Auswählen zumindest eines Autointerieurprodukts aus einer Vielzahl von in einer Datenbank hinterlegten Autointerieurprodukten für einen Autofahrer (4), umfassend die folgenden Verfahrensschritte:
Eingeben (30-1) von zumindest einem Eingabeparameter, insbesondere Autotyp, Einsatzbereich des Autos, gewünschte Härte der Sitzpolsterung, und/oder anatomische Besonderheiten des Autofahrers (4) durch eine Eingabevorrichtung (9) eines Mobilgeräts (6),
Aufnehmen (30-2) eines Fotos (14) von zumindest einem Körperteil des Autofahrers (4) durch eine Kamera (7) des Mobilgeräts (6),
Bestimmen (30-3) zumindest eines Körperparameters des Autofahrers (4) auf Basis des aufgenommenen Fotos (14) durch eine Steuerung (8-1),
Bestimmen (30-4) jeweils einer Produkteignung einer Mehrzahl von Autointerieurprodukten der Vielzahl von in der Datenbank hinterlegten Autointerieurprodukten in Abhängigkeit des bestimmten Körperparameters und in Abhängigkeit des zumindest einen eingegebenen Eingabeparameters durch die Steuerung (8-1),
Bestimmen (30-5) einer priorisierten Liste der Mehrzahl von Autointerieurprodukten durch die Steuerung (8-1), wobei in der priorisierten Liste die Priorität der jeweiligen Autointerieurprodukte mit zunehmender Produkteignung steigt, und
Ausgeben (30-6) einer Auswahl von zumindest einem Autointerieurprodukt für den Autofahrer (4) durch das Mobilgerät (6), wobei die Auswahl das zumindest eine Autointerieurprodukt der priorisierten Liste der Mehrzahl von Autointerieurprodukten umfasst, dessen jeweilige bestimmte Produkteignung eine geringste Differenz oder gar keine Differenz zu einer maximalen Produkteignung aufweist.

2. Verfahren (30) nach Anspruch 1, wobei das Ausgeben (30-6) das Ausgeben (30-6) einer Auswahl von mehreren Autointerieurprodukten für den Autofahrer (4) durch das Mobilgerät (6) umfasst, wobei die Auswahl die mehreren Autointerieurprodukte der priorisierten Liste der Mehrzahl von Autointerieurprodukten umfasst, deren jeweilige Differenz zwischen der jeweiligen Produkteignung und der maximalen Produkteignung geringer als ein Schwellenwert ist, wobei der Schwellenwert insbesondere weniger als 20%, 10%, 5%, 2% oder 1% der maximalen Produkteignung beträgt.

3. Verfahren (30) nach Anspruch 1 oder 2, wobei das Verfahren (30) das Auswählen zumindest eines Autositzes, insbesondere von zumindest einem Polsterpad eines Autositzes, aus einer Vielzahl von in einer Datenbank hinterlegten Autositzen, insbesondere von einer Vielzahl von in einer Datenbank hinterlegten Polsterpads, für einen Autofahrer (4) umfasst,
umfassend Eingeben (30-1) von zumindest einem Eingabeparameter, insbesondere Autotyp, Einsatzbereich des Autos, gewünschte Härte der Sitzpolsterung, und/oder anatomische Besonderheiten des Autofahrers (4) durch die Eingabevorrichtung (9) des Mobilgeräts (6),
umfassend Aufnehmen (30-2) eines Fotos (14) von zumindest einem Körperteil des Autofahrers (4) durch die Kamera (7) des Mobilgeräts (6),
wobei das Bestimmen (30-3) des zumindest eines Körperparameters des Autofahrers (4) das Bestimmen einer Schulterbreite (20-4, 20-4.1), eines Sitzknochenabstands (16), einer Kniehöhe (20-5), einer Hüftbreite (20-6), einer Schrittlänge (20-2) und/einer Körpergröße (20-3) des Autofahrers (4) auf Basis des aufgenommenen Fotos (14) von zumindest einem Körperteil des Autofahrers (4) durch die Steuerung (8-1) umfasst,
wobei das Bestimmen (30-4) der jeweiligen Produkteignung das Bestimmen jeweils einer Sitzeignung, insbesondere Padeignung, einer Mehrzahl von Autositzen, insbesondere Polsterpads, der Vielzahl von in der Datenbank hinterlegten Autositzen, insbesondere Polsterpads, in Abhängigkeit der bestimmten Schulterbreite (20-4, 20-4.1), Sitzknochenabstand (16), Kniehöhe (20-5), Hüftbreite (20-6), Schrittlänge (20-2) und/oder Körpergröße (20-3) und in Abhängigkeit des zumindest einen eingegebenen Eingabeparameters durch die Steuerung (8-1) umfasst,
wobei das Bestimmen (30-5) der priorisierten Liste das Bestimmen einer priorisierten Liste einer Mehrzahl von Autositzen, insbesondere Polsterpads, durch die Steuerung (8-1) umfasst, wobei in der priorisierten Liste die Priorität der jeweiligen Autositze, insbesondere Polsterpads mit zunehmender Sitzeignung, insbesondere Padeignung, steigt, und
wobei das Ausgeben (30-6) der Auswahl das Ausgeben von zumindest einem Autositz, insbesondere Polsterpad, für den Autofahrer (4) durch das Mobilgerät (6) umfasst, wobei die Auswahl den zumindest einen Autositz, insbesondere Polsterpad, der Mehrzahl von Autositzen, insbesondere Polsterpads, umfasst, dessen jeweilige bestimmte Sitzeignung, insbesondere Padeignung, die geringste Differenz oder gar keine Differenz zu einer maximalen Sitzeignung, insbesondere Padeignung, aufweist.

4. Verfahren (30) nach Anspruch 3, wobei das Aufnehmen (30-2) des Fotos (14) das Erfassen eines Gesäßabdrucks (3) des Autofahrers (4) durch eine Erfassungsvorrichtung (2), wobei der Gesäßabdruck (3) jeweils einen Druckbereich (12-1, 12-2) der beiden Sitzknochen des auf der Erfassungsvorrichtung (2) sitzenden Autofahrers (4) umfasst und das Aufnehmen (30-2) eines Fotos (14) des erfassten Gesäßabdrucks (3) durch die Kamera (7) des Mobilgeräts (6) umfasst, und wobei das Bestimmen (30-3) des zumindest einen Körperparameters das Bestimmen eines Sitzknochenabstands (16) des Autofahrers (4) auf Basis des aufgenommenen Fotos (14) des erfassten Gesäßabdrucks (3) durch die Steuerung (8-1) umfasst.

5. Verfahren (30) nach Anspruch 4, wobei die Erfassungsvorrichtung (2) eine Grundplatte mit Noppen umfasst, auf welchen ein Blatt Papier (11) angeordnet ist, wobei der Autofahrer (4) während des Erfassens des Gesäßabdrucks (3) mit seinem Gesäß auf dem Papier (11) sitzt, und wobei während des Erfassens des Gesäßabdrucks (3) die Noppen durch das Papier (11) hindurchgedrückt werden, um die Druckbereiche (12-1, 12-2) der beiden Sitzknochen des Autofahrers (4) auf dem Papier (11) zu erhalten, wobei insbesondere auf dem Blatt Papier (11) ein umlaufender Rahmen (13) angeordnet ist, und wobei das Bestimmen des Sitzknochenabstands (16) insbesondere das Erkennen und Rektifizieren sowie Ausrichten des aufgenommenen Fotos (14) des Gesäßabdrucks (3) auf Basis des umlaufenden Rahmens (13) durch eine Steuerung (8-1) umfasst.

6. Verfahren (30) nach Anspruch 4, wobei die Erfassungsvorrichtung (2) ein lichttransparentes mit Gel gefülltes Sitzkissen umfasst, wobei der Autofahrer (4) während des Erfassens des Gesäßabdrucks (3) mit seinem Gesäß auf dem Sitzkissen sitzt, und wobei während des Erfassens des Gesäßabdrucks (3) sich die Verteilung des Gels innerhalb des Sitzkissens verändert, um eine visuelle Darstellung der Druckbereiche (12-1, 12-2) der beiden Sitzknochen des Autofahrers (4) auf dem Sitzkissen zu erhalten.

7. Verfahren (30) nach einem der vorangehenden Ansprüche, wobei das Aufnehmen (30-2) eines Fotos (14) das Aufnehmen (30-2) eines Fotos (14) des gesamten Körpers des Autofahrers (4) durch die Kamera (7) des Mobilgeräts (6) umfasst, und wobei das Bestimmen (30-3) des zumindest einen Körperparameters das Bestimmen (30-3) der Körpergröße (20-3) mit den folgenden Verfahrensschritten umfasst:
Bestimmen eines obersten Punkts (4-2), insbesondere an dem Skalp, des Autofahrers (4) auf dem Foto (14) durch die Steuerung (8-1),
Bestimmen eines untersten Punkts (4-3), insbesondere an dem Mittelpunkt zwischen den beiden Fersen, des Autofahrers (4) auf dem Foto (14) durch die Steuerung (8-1), und
Bestimmen einer Differenz zwischen dem obersten Punkt (4-2) und dem untersten Punkt (4-3) durch die Steuerung (8-1), um die Körpergröße (20-3) des Autofahrers (4) zu bestimmen.

8. Verfahren (30) nach einem der vorangehenden Ansprüche, wobei das Aufnehmen (30-2) eines Fotos (14) das Aufnehmen (30-2) eines Fotos (14) des gesamten Körpers des Autofahrers (4) durch die Kamera (7) des Mobilgeräts (6) umfasst, und wobei das Bestimmen (30-3) des zumindest einen Körperparameters das Bestimmen der Schulterbreite von Gelenk zu Gelenk (20-4), der äußeren Schulterbreite (20-4.1), Sitzknochenabstand (16), Kniehöhe (20-5), Hüftbreite (20-6), und/oder Schrittlänge (20-2) mit den folgenden Verfahrensschritten umfasst:
Bestimmen eines obersten Punkts (4-2), insbesondere an dem Skalp, des Autofahrers (4) auf dem Foto (14) durch die Steuerung (8-1),
Bestimmen eines untersten Punkts (4-3), insbesondere an dem Mittelpunkt zwischen den beiden Fersen, des Autofahrers (4) auf dem Foto (14) durch die Steuerung (8-1),
Bestimmen einer Differenz zwischen dem obersten Punkt (4-2) und dem untersten Punkt (4-3) durch die Steuerung (8-1), um die Körpergröße (20-3) des Autofahrers (4) zu bestimmen, und
Multiplizieren der bestimmten Körpergröße (20-3) mit jeweils einem Faktor, wobei der jeweilige Faktor in Abhängigkeit von der Schulterbreite von Gelenk zu Gelenk (20-4) und/oder der äußeren Schulterbreite (20-4.1), von dem Sitzknochenabstand (16), von der Kniehöhe (20-5), von der Hüftbreite (20-6), und/oder von der Schrittlänge (20-2) festgelegt ist, um die Schulterbreite von Gelenk zu Gelenk (20-4) und/oder der äußeren Schulterbreite (20-4.1), den Sitzknochenabstand (16), die Kniehöhe (20-5), die Hüftbreite (20-6), und/oder die Schrittlänge (20-2) zu bestimmen.

9. Verfahren (30) nach einem der vorangehenden Ansprüche, wobei das Aufnehmen (30-2) eines Fotos (14) das Aufnehmen (30-2) eines Fotos (14) des gesamten Körpers des Autofahrers (4) durch die Kamera (7) des Mobilgeräts (6) umfasst, und wobei das Bestimmen (30-3) des zumindest einen Körperparameters das Bestimmen (30-3) der Schrittlänge (20-2) mit den folgenden Verfahrensschritten umfasst:
Bestimmen eines obersten Punkts (4-2), insbesondere an dem Skalp, des Autofahrers (4) auf dem Foto (14) durch die Steuerung (8-1),
Bestimmen eines untersten Punkts (4-3), insbesondere an dem Mittelpunkt zwischen den beiden Fersen, des Autofahrers (4) auf dem Foto (14) durch die Steuerung (8-1),
Bestimmen eines mittleren Punkts (4-4), insbesondere an der Hüfte, des Autofahrers (4) auf dem Foto (14) durch die Steuerung (8-1),
Bestimmen des Abstands (20-1) zwischen dem untersten Punkt (4-3) und dem mittleren Punkt (4-4) durch die Steuerung (8-1), und
Reduzieren des Abstands (20-1), um die Schrittlänge (20-2) des Autofahrers (4) zu bestimmen.

10. Verfahren (30) nach einem der Ansprüche, wobei das jeweilige Bestimmen (30-4) der Produkteignung, insbesondere Sitzeignung, insbesondere Padeignung, die folgenden Verfahrensschritte umfasst:
Zuordnen jeweils eines numerischen Wertes zu dem bestimmten zumindest einen Körperparameter, insbesondere Schulterbreite von Gelenk zu Gelenk (20-4) und/oder der äußeren Schulterbreite (20-4.1), Sitzknochenabstand (16), Kniehöhe (20-5), Hüftbreite (20-6), Schrittlänge (20-2) und/oder Körpergröße (20-3), und zu dem zumindest einen Eingabeparameter durch die Steuerung (8-1),
Gewichten der jeweils zugeordneten numerischen Werte durch die Steuerung (8-1), um gewichtete numerische Werte zu erhalten, und
Bilden eines Mittelwerts aus der Summe der gewichteten numerischen Werte, um die jeweilige Sitzeignung, insbesondere Padeignung zu erhalten.

11. Verfahren (30) nach einem der vorangehenden Ansprüche, wobei das Verfahren (30) das Auswählen zumindest eines Autolenkers, insbesondere von zumindest einem Griffstück eines Autolenkers, aus einer Vielzahl von in einer Datenbank hinterlegten Autolenkern, insbesondere von einer Vielzahl von in einer Datenbank hinterlegten Griffstücken, für einen Autofahrer (4) umfasst,
umfassend Eingeben (30-1) von zumindest einem Eingabeparameter, insbesondere Autotyp, Einsatzbereich des Autos, und/oder anatomische Besonderheiten des Autofahrers (4) durch eine Eingabevorrichtung (9) eines Mobilgeräts (6),
umfassend Aufnehmen (30-2) eines Fotos (14) von zumindest einem Körperteil des Autofahrers (4), insbesondere einer Hand (4-1) oder des gesamten Körpers des Autofahrers (4), durch eine Kamera (7) des Mobilgeräts (6),
wobei das Bestimmen (30-3) des zumindest einen Körperparameters des Autofahrers (4) das Bestimmen (30-3) einer Handlänge (17-1) und/oder einer Handbreite (17-2) einer Hand (4-1) des Autofahrers (4) und/oder das Bestimmen einer Körpergröße (20-3) des Autofahrers (4) auf Basis des aufgenommenen Fotos (14) durch die Steuerung (8-1) umfasst,
wobei das Bestimmen (30-4) der jeweiligen Produkteignung das Bestimmen (30-4) jeweils einer Lenkereignung, insbesondere Griffstückeignung, einer Mehrzahl von Autolenkern, insbesondere Griffstücken, der Vielzahl von in der Datenbank hinterlegten Autolenkern, insbesondere Griffstücken, in Abhängigkeit von der bestimmten Handlänge (17-1) und/oder Handbreite (17-2) und/oder Körpergröße (20-3) und in Abhängigkeit des zumindest einen eingegebenen Eingabeparameters durch die Steuerung (8-1) umfasst,
wobei das Bestimmen der priorisierten Liste das Bestimmen einer priorisierten Liste einer Mehrzahl von Autolenkern, insbesondere Griffstücken, durch die Steuerung (8-1) umfasst, wobei in der priorisierten Liste die Priorität der jeweiligen Autolenker, insbesondere Griffstücke mit zunehmender Lenkereignung, insbesondere Griffstückeignung, steigt, und
wobei das Ausgeben (30-6) der Auswahl das Ausgeben von zumindest einem Autolenker, insbesondere Griffstück, für den Autofahrer (4) durch das Mobilgerät (6) umfasst, wobei die Auswahl den zumindest einen Autolenker, insbesondere Griffstück, der Mehrzahl von Autolenkern, insbesondere Griffstücken, umfasst, dessen jeweilige bestimmte Lenkereignung, insbesondere Griffstückeignung, die geringste Differenz oder gar keine Differenz zu einer maximalen Lenkereignung, insbesondere Griffstückeignung, aufweist.

12. Verfahren (30) nach Anspruch 11, wobei das Aufnehmen (30-2) des Fotos (14) einer Hand (4-1) des Autofahrers (4) vor einem Referenzhintergrund (18) durchgeführt, wobei der Referenzhintergrund (18) ein Referenzsymbol (19) mit einer definierten Größe aufweist, und wobei das Bestimmen der Handlänge (17-1) und/oder der Handbreite (17-2) der Hand (4-1) des Autofahrers (4) auf Basis des Fotos (14) der Hand (4-1) und auf Basis der definierten Größe des Referenzsymbols (19) durchgeführt wird.

13. Verfahren (30) nach einem der Ansprüche 11 oder 12, wobei das Aufnehmen (30-2) eines Fotos (14) das Aufnehmen (30-2) eines Fotos (14) des gesamten Körpers des Autofahrers (4) durch die Kamera (7) des Mobilgeräts (6) umfasst, und wobei das Bestimmen der Körpergröße (20-3) die folgenden Verfahrensschritte umfasst:
Bestimmen eines obersten Punkts (4-2), insbesondere an dem Skalp, des Autofahrers (4) auf dem Foto (14) durch die Steuerung (8-1),
Bestimmen eines untersten Punkts (4-3), insbesondere an dem Mittelpunkt zwischen den beiden Fersen, des Autofahrers (4) auf dem Foto (14) durch die Steuerung (8-1), und
Bestimmen einer Differenz zwischen dem obersten Punkt (4-2) und dem untersten Punkt (4-3) durch die Steuerung (8-1), um die Körpergröße (20-3) des Autofahrers (4) zu bestimmen.

14. Verfahren (30) nach einem der Ansprüche 11 bis 13, wobei das Bestimmen der jeweiligen Lenkereignung, insbesondere Griffstückeignung die folgenden Verfahrensschritte umfasst:
Zuordnen jeweils eines numerischen Wertes zu der bestimmten Handbreite (17-2) und/oder zu der bestimmten Handlänge (17-1), und/oder zu der bestimmten Körpergröße (20-3), sowie zu dem zumindest einen Eingabeparameter durch die Steuerung (8-1),
Gewichten der jeweils zugeordneten numerischen Werte durch die Steuerung (8-1), um gewichtete numerische Werte zu erhalten, und
Bilden eines Mittelwerts aus der Summe der gewichteten numerischen Werte, um die jeweilige Lenkereignung, insbesondere Griffstückeignung, zu erhalten.

15. System (1) zum Auswählen zumindest eines Autointerieurprodukts aus einer Vielzahl von in einer Datenbank hinterlegten Autointerieurprodukten für einen Autofahrer (4), umfassend:
eine Eingabevorrichtung (9) eines Mobilgeräts (6), welche zum Eingeben (30-1) von zumindest einem Eingabeparameter, insbesondere Autotyp, Einsatzbereich des Autos, gewünschte Härte der Sitzpolsterung, und/oder anatomische Besonderheiten des Autofahrers (4) ausgebildet ist,
eine Kamera (7) des Mobilgeräts (6), welche ausgebildet ist, ein Foto (14) von zumindest einem Körperteil des Autofahrers (4) aufzunehmen, und
eine Steuerung (8-1), welche ausgebildet ist, auf Basis des aufgenommenen Fotos (14) zumindest einen Körperparameter des Autofahrers (4) zu bestimmen,
wobei die Steuerung (8-1) ausgebildet ist, jeweils eine Produkteignung einer Mehrzahl von Autointerieurprodukten der Vielzahl von in der Datenbank hinterlegten Autointerieurprodukten in Abhängigkeit des bestimmten Körperparameters und in Abhängigkeit des zumindest einen Eingabeparameters zu bestimmen,
wobei die Steuerung (8-1) ausgebildet ist, eine priorisierte Liste der Mehrzahl von Autointerieurprodukten zu bestimmen, wobei in der priorisierten Liste die Priorität der jeweiligen Autointerieurprodukte mit zunehmender Produkteignung steigt,
wobei das Mobilgerät (6) ausgebildet ist, eine Auswahl von zumindest einem Autointerieurprodukten für den Autofahrer (4) auszugeben, wobei die Auswahl das zumindest eine Autointerieurprodukt der Mehrzahl von Autointerieurprodukten umfasst, dessen jeweilige bestimmte Produkteignung die geringste Differenz oder gar keine Differenz zu einer maximalen Produkteignung aufweist.
